(19) Europäisches Patentamt
European Patent Office
Office européen des brevets

(11) EP 0 891 345 B1

(12) FASCICULE DE BREVET EUROPEEN

(45) Date de publication et mention de la délivrance du brevet: **16.08.2001 Bulletin 2001/33**

(21) Numéro de dépôt: **97919476.8**

(22) Date de dépôt: **03.04.1997**

(51) Int Cl.[7]: **C07D 307/58**, C07D 405/06, C07D 409/06, A61K 31/34, A61K 31/38, A61K 31/44

(86) Numéro de dépôt international: **PCT/FR97/00602**

(87) Numéro de publication internationale: **WO 97/37984 (16.10.1997 Gazette 1997/44)**

(54) **NOUVEAUX DERIVES DIARYLMETHYLIDENE FURANIQUES, LEURS PROCEDES DE PREPARATION ET LEURS UTILISATIONS EN THERAPEUTIQUE**

DIARYLMETHYLIDENEFURAN-DERIVATE, VERFAHREN ZU DEREN HERSTELLUNG UND IHRE VERWENDUNGEN IN THERAPIE

NOVEL FURAN DIARYLMETHYLIDENE DERIVATIVES, METHOD FOR THEIR PREPARATION AND THERAPEUTICAL USES THEREOF

(84) Etats contractants désignés:
**AT BE CH DE DK ES FI FR GB GR IE IT LI LU MC NL PT SE**
Etats d'extension désignés:
**AL LT LV RO SI**

(30) Priorité: **04.04.1996 FR 9604236**
**26.06.1996 FR 9607922**

(43) Date de publication de la demande:
**20.01.1999 Bulletin 1999/03**

(73) Titulaire: **LABORATOIRES UPSA**
**47000 Agen (FR)**

(72) Inventeurs:
- **NICOLAI, Eric**
  **F-92500 Rueil-Malmaison (FR)**
- **TEULON, Jean-Marie**
  **F-78170 La Celle-Saint-Cloud (FR)**

(74) Mandataire: **Hubert, Philippe**
**Cabinet Beau de Loménie**
**158, rue de l'Université**
**75340 Paris Cédex 07 (FR)**

(56) Documents cités:
**WO-A-94/15932**
**WO-A-95/00501**
**WO-A-96/08482**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne en tant que produits nouveaux, les dérivés diarylméthylidène furaniques de formule générale (I).

Une des voies de biotransformation de l'acide arachidonique est la voie de la cyclooxygénase ; elle permet la transformation de l'acide arachidonique en PGG2 puis en PGH2. Des travaux récents sur le clonage et le séquençage de la cyclooxygénase ont permis de mettre en évidence chez plusieurs espèces et chez l'homme en particulier, deux isoenzymes COX-1 et COX-2. La première est une enzyme constitutive, exprimée dans la plupart des tissus alors que la seconde qui est exprimée dans quelques tissus comme le cerveau, est inductible dans la majorité des tissus par de nombreux produits, en particulier par les cytokines et les médiateurs produits au cours de la réaction inflammatoire. Chaque enzyme joue un rôle différent et l'inhibition de COX-1 ou de COX-2 va provoquer des conséquences qui ne sont pas identiques . L'inhibition de COX-1 provoquera une diminution des prostaglandines participant à l'homéostasie ce qui peut entraîner des effets secondaires. L'inhibition de COX-2 provoquera une diminution des prostaglandines produites en situation d'inflammation. Ainsi l'inhibition sélective de COX-2 permet d'obtenir un agent anti-inflammatoire bien toléré.

Les composés de l'invention permettent d'obtenir cette inhibition sélective. En conséquence, les composés en question présentent un profil pharmacologique très intéressant dans la mesure où ils sont doués de propriétés anti-inflammatoires et antalgiques tout en étant remarquablement bien tolérés notamment au niveau gastrique. Ils seront particulièrement indiqués pour le traitement des phénomènes inflammatoires et pour le traitement de la douleur.

On peut citer par exemple, leur utilisation dans le traitement de l'arthrite, notamment l'arthrite rhumatoïde, la spondylarthrite, l'arthrite de la goutte, l'ostéoarthrite, l'arthrite juvénile, les maladies auto immunes, le lupus érythèmateux. Ils seront également indiqués pour le traitement de l'asthme bronchique, des dysménorrhées, des tendinites, des bursites, des inflammations dermatologiques telles que le psoriasis, l'eczéma, les brûlures, les dermatites. Ils peuvent également être utilisés pour le traitement des inflammations gastro intestinales, la maladie de Crohn, les gastrites, les colites ulcératives, la prévention du cancer, notamment l'adénocarcinome du colon, la prévention des maladies neurodégénératives particulièrement la maladie d'Alzheimer, la prévention du stroke, l'épilepsie et la prévention du travail utérin prématuré.

Leurs propriétés antalgiques permettent en outre leur utilisation dans tous les symptômes douloureux notamment dans le traitement des algies musculaires, articulaires ou nerveuses, des douleurs dentaires, des zonas et des migraines, dans le traitement des affections rhumatismales, des douleurs d'origine cancéreuse, mais aussi à titre de traitements complémentaires dans les états infectieux et fébriles.

La présente invention concerne également le procédé de préparation des dits produits et leurs applications en thérapeutique.

Certains dérivés sont décrits dans la littérature comme possédant des propriétés inhibitrices sélectives de la cyclooxygénase-2. On peut citer par exemple, les composés décrits dans les demandes de brevet suivantes :

WO 95 00501 A (Merck Frosst Canada Inc.)
WO 94 15932 A (G.D. Searle et Co.)
WO 96 08482 A (Merck et Co. Inc.)

D'une façon générale, la majorité des composés décrits dans ces documents comme inhibiteurs sélectifs de la cyclooxygénase-2, sont des dérivés d'hétérocycles à 5 atomes, substitués par deux noyaux aromatiques directement liés à l'hétérocycle et se trouvant sur deux carbones adjacents de cet hétérocycle.

La demanderesse a découvert, de façon surprenante, que des dérivés portant les deux noyaux aromatiques sur un même carbone, ces deux noyaux aromatiques étant reliés à l'hétérocycle non pas directement mais par l'intermédiaire d'une double liaison, possèdent des propriétés inhibitrices sélectives de la cyclo oxygénase-2 remarquables.

Ces dérivés diarylméthylidène furaniques sont caractérisés en ce qu'ils répondent à la formule générale (I):

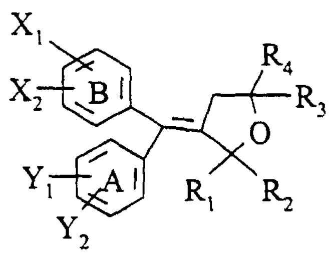

Formule (I)

dans laquelle :

les cycles A et B représentent indépendamment :

- un radical phényle
- un radical naphtyle
- un radical dérivé d'un hétérocycle comportant 5 à 6 chaînons et possédant de 1 à 4 hétéroatomes
- un radical dérivé d'un cycle hydrocarboné saturé ayant de 3 à 7 atomes de carbone

au moins l'un des substituants $X_1$, $X_2$, $Y_1$ ou $Y_2$ représente obligatoirement :

- un groupement $-S(O)_n-R$ dans lequel n est un nombre entier égal à 0,1 ou 2 et R est un radical alkyle inférieur de 1 à 6 atomes de carbone, un radical halogéno alkyle inférieur de 1 à 6 atomes de carbone,
- un groupement $-SO_2NH_2$;
  et se trouve en position para, sur le radicalphényle représenté par A ou B

les autres représentent indépendamment

- l'atome d'hydrogène
- un atome d'halogène
- un radical alkyle inférieur de 1 à 6 atomes de carbone
- un radical trifluorométhyle,
- un radical O-alkyle inférieur de 1 à 6 atomes de carbone

ou encore

$X_1$ et $X_2$ ou $Y_1$ et $Y_2$ représentent un groupement méthylène dioxy,
$R_1$, $R_2$, $R_3$ et $R_4$ représentent indépendamment

- l'atome d'hydrogène
- un atome d'halogène
- un radical alkyle inférieur de 1 à 6 atomes de carbone,
- un radical halogénoalkyle inférieur de 1 à 6 atomes de carbone,
- un radical aromatique choisi dans le groupe comprenant phényle, naphtyle, thiényle, furyle ou pyridyle,

ou encore

$R_1R_2$ ou $R_3R_4$ représentent un atome d'oxygène
ou bien $R_1$, $R_2$ ou $R_3$, $R_4$, forment ensemble avec l'atome de carbone auquel ils sont attachés un cycle hydrocarboné saturé ayant de 3 à 7 atomes de carbone.

Dans la description et les revendications, on entend par alkyle inférieur une chaîne hydrocarbonée ayant de 1 à 6 atomes de carbone, linéaire ou ramifiée. Un radical alkyle inférieur est par exemple un radical méthyle, éthyle, propyle, isopropyle, butyle, isobutyle, tertiobutyle, pentyle, isopentyle, hexyle, isohexyle.
Par radical halogénoalkyle inférieur, on entend un radical alkyle de 1 à 6 atomes de carbone dont 1 à 7 atomes d'hydrogène ont été substitués par 1 à 7 atomes d'halogène. Un radical halogénoalkyle inférieur est par exemple un

radical trifluorométhyle, un radical 2,2,2-trifluoroéthyle, un radical pentafluoroéthyle, un radical 2,2-difluoro-3,3,3-trifluoro propyle, un radical heptafluoropropyle, un radical chlorométhyle ou bromométhyle.

On entend par halogène un atome de chlore, de brome, d'iode ou de fluor.

Par cycle hydrocarboné saturé ayant de 3 à 7 atomes de carbone, on entend désigner cyclopropane, cyclobutane, cyclopentane, cyclohexane ou cycloheptane.

Par un radical dérivé d'un hétérocycle, on entend tous cycles aromatiques comportant dans le noyau de un à quatre hétéroatomes : azote, oxygène ou soufre. Parmi ces cycles, on préférera particulièrement la pyridine, le furane, le thiophène et également le pyrrole, l'imidazole, le pyrazole, la pyrazine, la pyrimidine, la pyridazine, un oxazole, un oxadiazole, un thiazole, un thiadiazole.

Les dérivés de formule (I) précités, peuvent présenter des centres d'asymétrie et/ou se trouver sous la forme de dérivés cis ou trans. L'invention couvre les racémiques, les mélanges de composés cis et trans mais également les produits optiquement actifs, les dérivés cis et les dérivés trans pris indépendamment. L'obtention de ces produits purs sera réalisée selon les méthodes connues de l'homme de l'art, en particulier par chromatographie notamment sur des colonnes chirales lorsqu'il s'agira d'isomères optiques. Cette séparation peut se faire également dans certains cas par simple recristallisation. Elle peut être réalisée soit sur le produit final, soit sur un intermédiaire de synthèse, dans ce cas la suite de la synthèse respectera la stéréochimie de la molécule intermédiaire.

Avantageusement, les dérivés conformes à l'invention sont les dérivés de formule (I) précitée dans laquelle :

les cycles A et B représentent indépendamment un radical :

- phényle
- naphtyle
- pyridyle
- furyle
- thiényle
- cyclohexyle

au moins l'un des substituants $X_1$, $X_2$, $Y_1$ ou $Y_2$ représente obligatoirement un groupement $SCH_3$, $SO_2CH_3$ ou $SO_2NH_2$,
les autres représentent indépendamment:

- l'atome d'hydrogène
- un atome d'halogène
- un radical alkyle inférieur de 1 à 6 atomes de carbone
- un radical trifluorométhyle
- un radical 0-alkyle inférieur de 1 à 6 atomes de carbone

$R_1R_2$ représentent un atome d'oxygène
$R_3$,$R_4$ représentent indépendamment l'atome d'hydrogène ou un radical alkyle inférieur de 1 à 6 atomes de carbone.

Avantageusement, dans le cadre de la présente invention, on utilisera un composé de formule (I) dans laquelle, l'une au moins des conditions suivantes est réalisée :

- le cycle B représente un radical phényle
- $X_1$ représente le groupement 4-$SO_2CH_3$ ou le groupement 4-$SO_2NH_2$
- $X_2$ représente l'atome d'hydrogène
- le cycle A représente un radical phényle ou pyridyle
- $Y_1$ représente l'atome de fluor, l'atome de chlore ou un radical méthyle
- $Y_2$ représente l'atome d'hydrogène, l'atome de fluor ou l'atome de chlore
- $R_1R_2$ représente un atome d'oxygène
- $R_3$ représente l'atome d'hydrogène
- $R_4$ représente l'atome d'hydrogène

Les composés de l'invention particulièrement préférés sont les dérivés de formule :

(E)-3-[1-(4-fluorophényl)-1-(4-méthanesulfonylphényl)méthylidène]-dihydrofuran-2-one

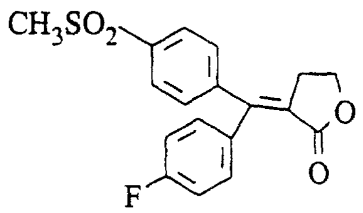

(Z)-3-[1-(4-chlorophényl)-1-(4-méthanesulfonylphényl)méthylidène]-dihydrofuran-2-one

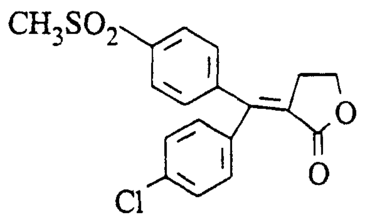

(Z)-3-[1-(3,4-dichlorophényl)-1-(4-méthanesulfonylphényl)méthylidène]-dihydrofuran-2-one

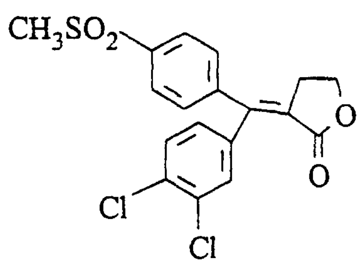

(Z)-3-[1-(6-chloropyridin-3-yl)-1-(4-méthanesulfonylphényl)méthylidène]-dihydro-furan-2-one

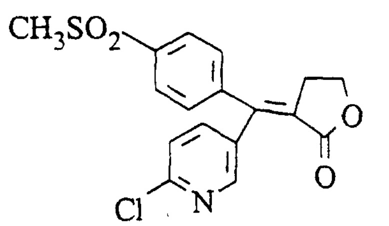

(Z)-4-[(4-chlorophényl)-(2-oxo-dihydro-furan-3-ylidène)-méthyl]-benzène sulfonamide

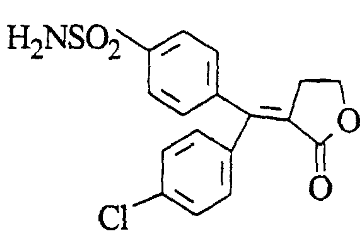

(Z)-4-[(3-fluoro-4-méthylphényl)-(2-oxo-dihydro-furan-3-ylidène)-méthyl]-benzènesulfonamide

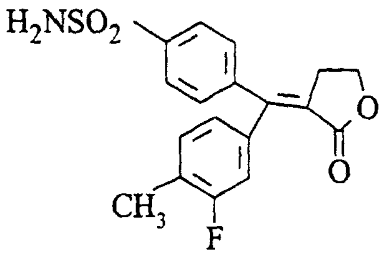

Selon l'invention, les composés de formule (I) peuvent être synthétisés de la façon suivante :

Par une réaction de Friedel et Crafts du chlorure d'acide de formule (II)

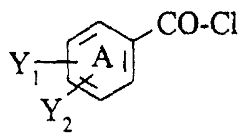

Formule (II)

dans laquelle A, $Y_1$ et $Y_2$ sont définis comme ci-dessus sur le thioanisole, on obtiendra la cétone de formule (III)

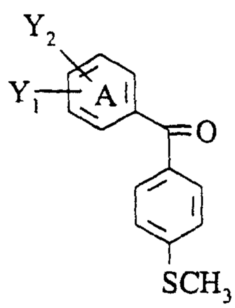

Formule (III)

dans laquelle A, $Y_1$ et $Y_2$ sont définis comme ci-dessus. Cette cétone peut également être obtenue par une réaction de Grignard : réaction du magnésien d'un dérivé bromoaromatique qui peut être substitué, sur le p-méthylthio benzonitrile.

Par traitement de cette cétone par un agent oxydant comme par exemple l'acide métachloroperbenzoïque, le perborate de sodium ou l'eau oxygénée en présence d'une quantité catalytique de sels de molybdène, on obtiendra le dérivé de formule (IV)

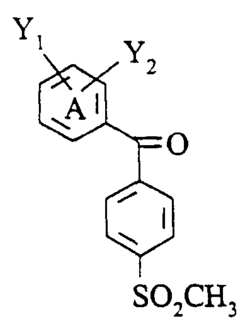

Formule (IV)

dans laquelle A, $Y_1$ et $Y_2$ sont définis comme ci-dessus. Par traitement du dérivé de formule (IV) par une réaction de Reformatsky modifiée avec une bromobutyrolactone de formule (V)

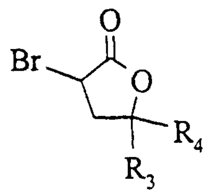

Formule (V)

dans laquelle $R_3$ et $R_4$ sont définis comme ci-dessus, en présence de magnésium et d'une faible quantité d'iodure de méthyle pour amorcer la réaction, on obtiendra les dérivés de formule (VI)

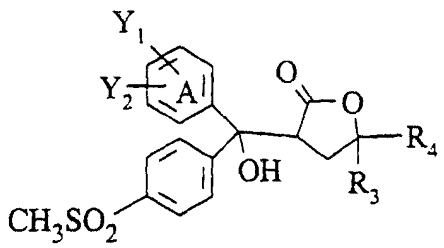

Formule (VI)

dans laquelle A, $Y_1$, $Y_2$, $R_3$ et $R_4$ sont définis comme ci-dessus. Enfin, par déshydratation des dérivés de formule (VI) par chauffage dans le toluène par exemple en présence d'acide paratoluènesulfonique ou par traitement par l'anhydride trifluoroacétique dans l'acide trifluoroacétique on obtiendra les composés de formule (I):

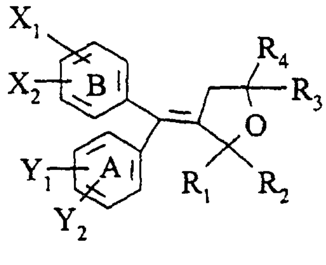

Formule (I)

dans laquelle B représente un noyau phényl, $X_1$ représente un groupement 4-$SO_2CH_3$, $X_2$ représente l'atome d'hydrogène, $R_1R_2$ l'atome d'oxygène et A, $Y_1$, $Y_2$, $R_3$ et $R_4$ sont définis comme ci-dessus.

Une variante de préparation consiste à traiter le produit de formule (III) soit par le dérivé de formule (V) selon une méthode identique de réaction de Reformatsky modifiée en présence de magnésium et d'iodure de méthyle pour amorcer la réaction, soit par des lactones de formule (V')

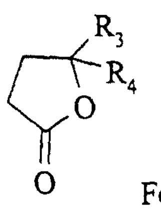

Formule (V')

dans laquelle, $R_3$ et $R_4$ sont définis comme ci-dessus, en présence de bromure de N,N-diéthylamino magnésium, préparé par action de la N,N- diéthylamine sur le bromure d'éthyl magnésium, selon la référence : K. Sisido, H. Nozaki, O. Kurihara J. Am. Chem. Soc., 74, 6254(1952) pour conduire soit aux composés déjà déshydratés de formule (I), soit aux composés de formule (VI')

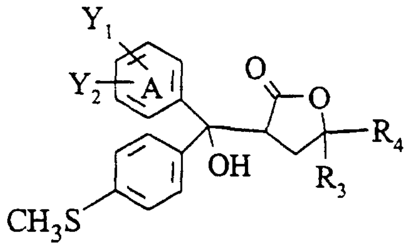

Formule (VI')

dans laquelle A, $Y_1$, $Y_2$, $R_3$ et $R_4$ sont définis comme ci-dessus. Les composés de formule (VI') seront alors déshydratés par traitement par l'anhydride trifluoroacétique et l'acide trifluoroacétique pour donner les composé de formule (I) ci-dessous :

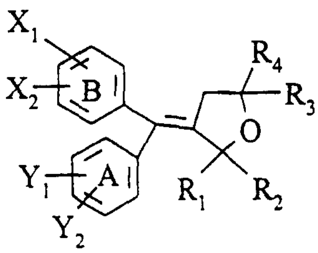

Formule (I)

dans laquelle B représente un noyau phényl, $X_1$ représente un groupement 4-$SCH_3$, $X_2$ représente l'atome d'hydrogène, $R_1R_2$ l'atome d'oxygène et A, $Y_1$, $Y_2$, $R_3$ et $R_4$ sont définis comme ci-dessus.

Le traitement du composé ainsi obtenu selon cette variante, par l'acide méta chloroperbenzoïque ou par un autre oxydant comme $NaBO_3$, 4 $H_2O$ conduira selon la quantité d'oxydant utilisée aux composés de formule (I)

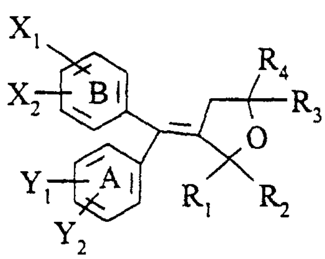

Formule (I)

dans laquelle B représente un noyau phényl, $X_1$ représente un groupement 4-$SOCH_3$ pour un équivalent d'oxydant ou un groupement 4-$SO_2CH_3$ pour deux équivalents d'oxydant, $X_2$ représente l'atome d'hydrogène, $R_1R_2$ l'atome d'oxygène et A, $Y_1$, $Y_2$, $R_3$ et $R_4$ sont définis comme ci-dessus.

Une autre variante de préparation de certains composés de formule (I) consiste à traiter une cétone de formule (III) par le succinate d'éthyle selon la réaction de Stobbe dans le t-butanol en présence de t-butylate de sodium ou de potassium par exemple, pour conduire aux composés de formule (VII)

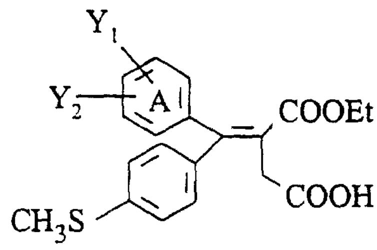

Formule (VII)

dans laquelle A, $Y_1$ et $Y_2$ sont définis comme ci-dessus. La réduction sélective de la fonction ester par exemple au borohydrure de calcium obtenu in situ à partir de borohydrure de potassium et du chlorure de calcium dans l'éthanol ou au diéthyl dihydroaluminate de sodium dans l'éther éthylique conduira après lactonisation des hydroxy acides obtenus aux dérivés de formule (VIII).

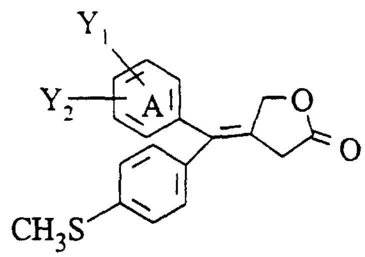

Formule (VIII)

dans laquelle A, $Y_1$ et $Y_2$ sont définis comme ci-dessus.

Les dérivés de formule (VIII) peuvent être oxydés comme précédemment décrit, le groupement $SCH_3$ étant alors transformé en groupement $SOCH_3$ ou $SO_2CH_3$ selon la quantité d'agent oxydant utilisée, pour conduire aux composés de formule (I)

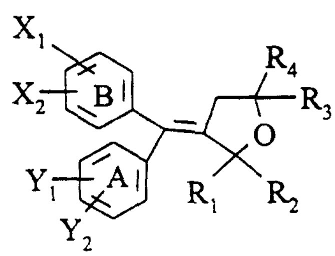

Formule (I)

dans laquelle B représente le noyau phényl, $X_1$ représente le groupement 4-$SOCH_3$ ou le groupement 4-$SO_2CH_3$, $X_2$, $R_1$ et $R_2$ représentent l'atome d'hydrogène, $R_3R_4$ l'atome d'oxygène et A, $Y_1$, $Y_2$, sont définis comme ci-dessus.

La réduction des dérivés de formule (VII) par l'hydrure double d'aluminium lithium dans le tétrahydrofurane par exemple, conduira aux diols de formule (IX)

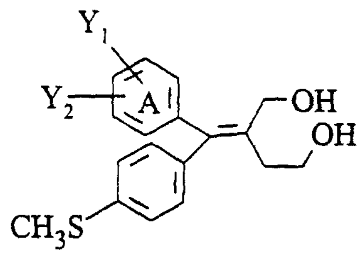

Formule (IX)

dans laquelle A, $Y_1$ et $Y_2$ sont définis comme ci-dessus.

La déshydratation de ces diols par l'acide sulfurique ou par un traitement au reflux du toluène en présence d'acide paratoluènesulfonique avec un appareil de Dean Stark, permettra d'obtenir les composés de formule (I)

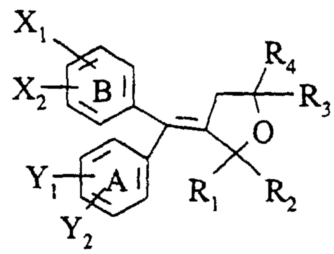

Formule (I)

dans laquelle A, $Y_1$ et $Y_2$ sont définis comme ci-dessus, B représente le noyau phényl, $X_1$ représente le groupement 4-$SCH_3$, $X_2$, $R_1$, $R_2$, $R_3$, $R_4$ représentent l'atome d'hydrogène.

Par traitement de ces dérivés avec un agent oxydant comme décrit précédemment on obtiendra les dérivés correspondants où $X_1$ représente le groupement 4-$SOCH_3$ ou 4-$SO_2CH_3$ selon la quantité d'oxydant utilisée.

D'autres variantes de préparation des composés de formule I peuvent être utilisées.

La réaction des cétones, composés de formule (IV) avec le succinate d'éthyle selon la réaction de Stobbe dans le t-butanol en présence de t-butylate de sodium ou de potassium par exemple, conduira aux composés de formule (X) :

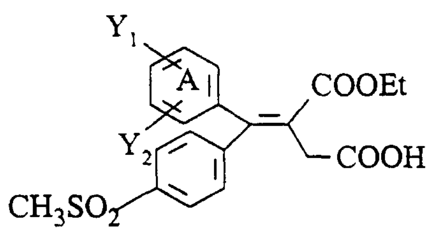

Formule (X)

dans laquelle A, $Y_1$ et $Y_2$ sont définis comme ci-dessus.

La réduction sélective cette fois, de la fonction acide, par exemple par action du borane ou du complexe borane / sulfure de méthyle dans le tétrahydrofurane ou l'éther éthylique, conduira aux alcools-esters de Formule (XI) :

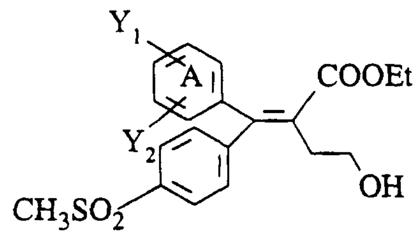

Formule (XI)

dans laquelle A, $Y_1$ et $Y_2$ sont définis comme ci-dessus.

Ces alcools esters de formule (XI), ou bien les alcools acides correspondants obtenus par l'hydrolyse de la fonction ester par la soude dans l'éthanol au reflux, seront cyclisés par chauffage dans un solvant aromatique tel que le toluène par exemple, en présence d'acide para-toluènesulfonique pour obtenir les composés de formule (I):

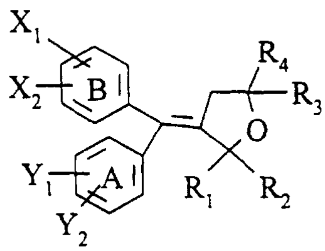

Formule (I)

dans laquelle A, $Y_1$, $Y_2$ sont définis comme ci-dessus, B représente le noyau phényl, $X_1$ représente le groupement 4-$SO_2CH_3$, $X_2$, $R_3$ et $R_4$ représentent l'atome d'hydrogène et $R_1R_2$ représente l'atome d'oxygène.

D'une façon analogue, on pourra préparer les composés de Formule (XII) dans laquelle A, $Y_1$, $Y_2$ ont la même signification que ci-dessus,selon le schéma réactionnel suivant dans lequel Ph représente un groupement phényl et Z représente un radical MgBr lorsque A est un noyau phényl et Li lorsque A est un noyau pyridyl.

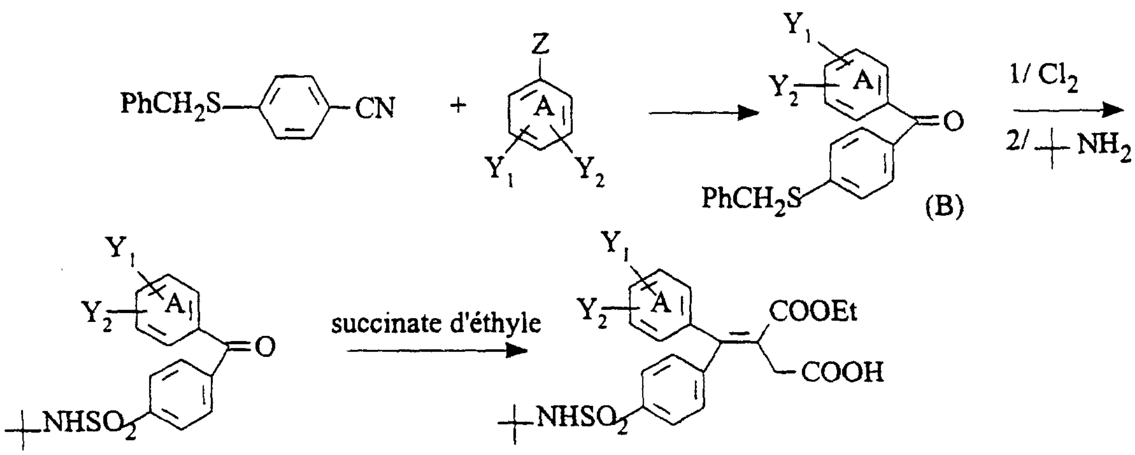


Formule (XII)

Une variante consiste à préparer la diarylcétone (B) utilisée dans le schéma réactionnel précédent, par action du

benzylmercaptan $PhCH_2SH$ sur une fluoro diarylcétone dans le diméthylformamide en présence d'hydrure de sodium ou de carbonate de sodium :

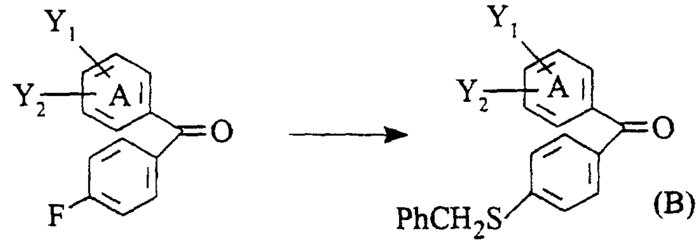

Les composés de formule (XII) seront traités comme les composés de formule (X) pour conduire aux composés de formule (I)

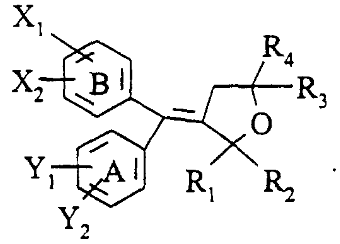

Formule (I)

dans laquelle B représente le noyau phényle, $X_1$ représente le groupe 4-$SO_2$NHt-Bu, $X_2$, $R_3$ et $R_4$ représentent l'atome d'hydrogène, $R_1R_2$ représente l'atome d'oxygène et A, $Y_1$ et $Y_2$ sont définis comme ci-dessus.

Par traitement de ces dérivés par un acide fort comme par exemple l'acide sulfurique concentré, l'acide trifluoroacétique ou par chauffage dans le toluène en présence d'acide para toluènesulfonique, on obtiendra les composés de formule (I) dans laquelle B représente le noyau phényle, $X_1$ représente le groupe 4-$SO_2NH_2$, $X_2$, $R_3$ et $R_4$ représentent l'atome d'hydrogène, $R_1R_2$ représente l'atome d'oxygène et A, $Y_1$ et $Y_2$ sont définis comme ci-dessus.

Les composés de formule (I) tels que définis ci-dessus sont des inhibiteurs de cyclooxygénase-2 et sont doués d'une très bonne activité anti-inflammatoire et analgésique associée à une excellente tolérance en particulier gastrique.

Ces propriétés justifient leur application en thérapeutique et l'invention a également pour objet, à titre de médicaments, les produits tels que définis par la formule (I) ci-dessus.

Ainsi, l'invention couvre également une composition pharmaceutique, caractérisée en ce qu'elle comprend une quantité pharmaceutiquement efficace d'au moins un composé de formule (I) tel que précédemment défini éventuellement incorporé dans un excipient, véhicule ou support pharmaceutiquement acceptable.

Ces compositions peuvent être administrées par voie buccale, rectale, par voie parentérale, par voie transdermique, par voie oculaire, par voie nasale ou par voie auriculaire.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les systèmes transdermiques, les collyres, les aérosols et sprays et les gouttes auriculaires. Elles sont préparées selon les méthodes usuelles. Le principe actif, constitué par une quantité pharmaceutiquement efficace d'au moins un composé de formule (I) défini comme ci-dessus peut y être incorporé à des excipients habituellement employés dans ces compositions pharmaceutiques, tels que le talc, la gomme arabique, le lactose, l'amidon, le stéarate de magnésium, la polyvidone, les dérivés de la cellulose, le beurre de cacao, les glycérides semi-synthétiques, les véhicules aqueux ou non, les corps gras d'origine animale ou végétale, les glycols, les divers agents mouillants, dispersants ou émulsifiants, les gels de silicone, certains polymères ou copolymères, les conservateurs, arômes et colorants.

L'invention couvre encore une composition pharmaceutique à activité anti-inflammatoire et antalgique permettant notamment de traiter favorablement les phénomènes inflammatoires et la douleur caractérisée en ce qu'elle comprend une quantité pharmaceutiquement efficace d'au moins un composé de formule (I) précitée dans un excipient,

véhicule ou support pharmaceutiquement acceptable. Selon un mode de réalisation, on prépare une composition pharmaceutique à activité anti-inflammatoire et antalgique permettant notamment de traiter favorablement les différentes inflammations et la douleur.

Selon une variante de réalisation, on prépare une composition formulée sous forme de gélules ou de comprimés dosés de 1 mg à 1000 mg ou sous forme de préparations injectables dosées de 0,1 mg à 500 mg. On pourra également utiliser des formulations sous forme de suppositoires, pommades, crèmes, gels, des préparations en aérosols, des préparations transdermiques ou des emplâtres.

L'invention couvre encore un procédé de traitement thérapeutique des mammifères, caractérisé en ce qu'on administre à ce mammifère une quantité thérapeutiquement efficace d'au moins un composé de formule (I) telle que précédemment définie. Selon une variante de réalisation de ce procédé de traitement. le composé de formule (I), soit seul, soit en association avec un excipient pharmaceutiquement acceptable, est formulé en gélules ou en comprimés dosés de 1 mg à 1000 mg pour l'administration par voie orale, ou sous forme de préparations injectables dosées de 0,1 mg à 500 mg ou encore sous forme de suppositoires, pommades, crèmes, gels ou de préparations en aérosols.

Ce procédé permet notamment de traiter favorablement les phénomènes inflammatoires et la douleur.

En thérapeutique humaine et animale, les composés de formule (I) peuvent être administrés seuls ou en association avec un excipient physiologiquement acceptable sous forme quelconque, en particulier par voie orale sous forme de gélules ou de comprimés ou par voie parentérale sous forme de soluté injectable. D'autres formes d'administration comme suppositoires, pommades, crèmes, gels ou des préparations en aérosols peuvent être envisagées.

Comme il ressortira clairement des essais de pharmacologie donnés en fin de description, les composés selon l'invention peuvent être administrés en thérapeutique humaine dans les indications précitées par voie orale sous forme de comprimés ou gélules dosés de 1 mg à 1000 mg ou par voie parentérale sous forme de préparations injectables dosées de 0,1 mg à 500 mg en une ou plusieurs prises journalières pour un adulte de poids moyen 60 à 70 kg.

En thérapeutique animale la dose journalière utilisable se situe entre 0,1 mg et 100 mg par kg.

D'autres caractéristiques et avantages de l'invention seront mieux compris à la lecture qui va suivre de quelques exemples nullement limitatifs, mais donnés à titre d'illustration.

**Exemple 1 : 4-fluoro-4'-méthylthiobenzophénone**

Formule (III) : A = phényl, $Y_1$ = 4-F, $Y_2$ = H

A une solution de 70 g (0.564 mole) de thioanisole et de 90.2 g (0.654 mole) de chlorure de 4-fluorobenzoyle dans 500 ml de dichlorométhane sont ajoutés par portion 86.4 g de trichlorure d'aluminium à une température comprise entre 0°C et 5°C. Après la fin de l'addition le mélange est ramené à température ambiante puis chauffé au reflux pendant 2 heures. Après refroidissement, le milieu réactionnel est coulé sur un mélange glace / acide chlorhydrique dilué et la phase organique est décantée puis séchée sur sulfate de magnésium et évaporée sous vide pour donner un résidu qui cristallise dans l'éther isopropylique et donne 118 g de 4-fluoro-4'-méthylthiobenzophénone de point de fusion 88°C.

**Exemple 2 : 4-Fluoro-4'-méthanesulfonylbenzophénone**

Formule (IV) : A = phényl, $Y_1$ = 4-F, $Y_2$ = H

A une solution de 25 g (0.1015 mole) de 4-fluoro-4'-méthylthio benzophénone, préparé à l'exemple 1, dans 350 ml de dichlorométhane sont ajoutés par portions 87 g d'acide 3-chloro perbenzoïque à 70 %, à une température comprise entre 0°C et 5°C. Le mélange est ensuite agité à 0°C pendant 30 minutes puis ramené à température ambiante et agité pendant 2 heures 30 minutes. Le précipité obtenu est essoré et lavé avec une solution de soude diluée puis dissout dans du dichlorométhane. La phase organique ainsi obtenue est séchée sur sulfate de magnésium et évaporée sous vide pour donner une huile qui cristallise dans l'éther isopropylique et donne 24.6 g de 4-fluoro-4'-méthanesulfonyl benzophénone de point de fusion 136°C.

**Exemple 3 : 3-[1-(4-fluorophényl)-1-hydroxy-1-(4-méthanesulfonyl phényl)méthyl]-dihydro-furan-2-one**

Formule (VI) : A=phényl, $Y_1$ = 4-F, $Y_2$ = H, $R_3$ = $R_4$ = H

Du magnésium en copeaux (3.5 g) est recouvert par du tétrahydrofurane anhydre et quelques gouttes de iodométhane sont ajoutés. Dès que la réaction a démarré, un mélange de 24.6 g de 4-fluoro-4'-méthanesulfonylbenzophénone et 8.1 ml d'∝-bromo-γ-butyrolactone dans 250 ml de tétrahydrofurane anhydre est coulé goutte à goutte de manière à entretenir un léger reflux. A la fin de l'addition, le milieu réactionnel est refroidi puis coulé sur un mélange de glace et d'acide sulfurique dilué à 10 %. La phase organique est extraite avec du tétrahydrofurane et lavée avec une solution saturée de bicarbonate de sodium puis séchée sur sulfate de magnésium. Après évaporation du solvant, le résidu obtenu est chromatographié sur gel de silice avec un mélange dichlorométhane 9 / acétone 1 pour donner 7 g de 3-[1-(4-fluorophényl)-1-hydroxy-1-(4-méthanesulfonyl phényl)méthyl]-dihydro-furan-2-one sous forme d'une poudre beige amorphe utilisée telle quelle pour la suite.

**Exemple 4 : (E)-3-[1-(4-fluorophényl)-1-(4-méthanesulfonylphényl) méthylidène]-dihydro-furan-2-one**

Isomère (E) : Formule (I) : A = B = phényl,

$Y_1$ = 4-F, $X_2$ = $Y_2$ = H, $X_1$ = 4-$SO_2CH_3$,

$R_1R_2$ = O, $R_3$ = $R_4$ = H

A une solution de 7 g de 3-[1-(4-fluorophényl)-1-hydroxy-1-(4-méthanesulfonylphényl)méthyl]-dihydro-furan-2-one préparé à l'exemple 3, dans 100 ml de toluène, sont ajoutés quelques mg d'acide 4-toluène sulfonique et le mélange est chauffé au reflux pendant 10 heures avec un Dean-Stark. Le solvant est ensuite évaporé à sec sous vide et le résidu est chromatographié dans un mélange dichlorométhane 9 / acétone 1 comme éluant pour donner une huile qui est chromatographiée dans le t-butyl méthyl éther comme éluant pour donner 2.9 g de (E)-3-[1-(4-fluorophényl)-1-(4-méthanesulfonylphényl) méthylidène]-dihydrofuran-2-one (deuxième produit élué) sous forme de cristaux de point de fusion 187-9°C. On récupère 1.5 g de (Z)-3-[1-(4-fluorophényl)-1-(4-méthanesulfonyl phényl) méthylidène]-dihydro-furan-2-one (premier produit élué) sous forme de cristaux de point de fusion 157-158°C.

**Exemple 5 : 4-chloro-4'-méthylthiobenzophénone**

Formule (III): A = phényl, $Y_1$ = 4-Cl, $Y_2$ = H

Préparé selon le mode opératoire de l'exemple 1.
Cristaux de point de fusion 134°C.

**Exemple 6 : 3-[1-(4-chlorophényl)-1-hydroxy-1-(4-méthylthiophényl) méthyl]-dihydro-furan-2-one**

Formule (VI') : A = phényl, $Y_1$ = 4-Cl, $Y_2$ = H, $R_3$ = $R_4$ = H

A 5,9 g de magnésium en copeaux recouverts de tétrahydrofurane anhydre sont ajoutés quelques gouttes de iodométhane. Dès que la réaction démarre, un mélange de 17 g de 4-chloro-4'-méthylthiobenzophénone et 12.6 ml d'∝-bromo-γ-butyrolactone dans 300 ml de tétrahydrofurane anhydre est ajouté goutte à goutte de façon à maintenir un léger reflux. Après la fin de l'addition, le mélange est agité à température ambiante pendant 1 heure 30 minutes

puis refroidi par un bain de glace. Une solution saturée de chlorure d'ammonium est ensuite ajoutée et le mélange est agité, puis décanté. La phase organique est séchée sur sulfate de magnésium et évaporée sous vide pour donner un résidu huileux qui après chromatographie sur gel de silice dans le dichlorométhane donne 8.5 g de 3-[1-(4-chlorophényl)-1-hydroxy-1-(4-méthylthiophényl) méthyl]-dihydro-furan-2-one sous forme d'huile utilisée brute pour la suite.

**Exemple 7 : 3-[1-(4-chlorophényl)-1-(4-méthylthiophényl)méthylidène]-dihydro-furan-2-one**

Formule (I) : A = B = phényl, $Y_1$ = 4-Cl, $Y_2$ = $X_1$ = H,

$X_2$ = 4-$SCH_3$, $R_1$ $R_2$ = O, $R_3$ = $R_4$ = H

A une solution de 8.6 g de 3-[1-(4-chlorophényl)-1-hydroxy-1-(4-méthylthiophényl) méthyl]-dihydro-furan-2-one préparé à l'exemple 6, dans 100 ml de dichlorométhane sont ajoutés 5.2 g d'anhydride trifluoroacétique et 3.8 ml d'acide trifluoroacétique. Le mélange est agité à température ambiante pendant 4 heures puis dilué à l'eau et décanté. La phase organique est séchée sur sulfate de magnésium et évaporée sous vide pour donner 7.5 g de 3-[1-(4-chlorophényl)-1-(4-méthylthiophényl)méthylidène]-dihydro-furan-2-one sous forme d'huile utilisée telle quelle pour la suite.

**Exemple 8 : (Z)-3-[1-(4-chlorophényl)-1-(4-méthanesulfonylphényl) méthylidène)- dihydro-furan-2-one**

Isomère (Z) : Formule (I) : A = B = phényl, $Y_1$ = 4-Cl,

$Y_2$ = $X_1$ = H, $X_2$ = 4-$SO_2CH_3$, $R_1R_2$ = O, $R_3$ = $R_4$ = H

A une solution de 9.5 g de 3-[1-(4-chlorophényl)-1-(4-méthylthiophényl) méthylidène]-dihydro-furan-2-one préparé à l'exemple 7, dans 120 ml d'acide acétique, sont ajoutés 11 g de perborate de sodium trihydraté. Le mélange est chauffé à 40-50°C pendant 5 heures puis refroidi. Les cristaux formés sont essorés, lavés à l'eau puis chromatographiés sur gel de silice dans un mélange dichlorométhane / acétone (99/1) pour donner 4.1 g de (Z)-3-[1-(4-chlorophényl)-1-(4-méthanesulfonylphényl) méthylidène]- dihydro-furan-2-one (1er produit élué) sous forme de cristaux de point de fusion 197-199°C.

L'isolation du deuxième produit élué permet d'obtenir 2.5 g de (E)-3-[1-(4-chlorophényl)-1-(4-méthanesulfonylphényl)méthylidène]-dihydro-furan-2-one sous forme de cristaux de point de fusion 211-212°C.

**Exemple 9 : 3-fluoro-4'-méthylthiobenzophénone**

Formule (III) : A = phényl, $Y_1$ = 3-F, $Y_2$ = H

Préparé selon le mode opératoire de l'exemple 1.

Cristaux de point de fusion 76°C.

**Exemple 10 : 3-fluoro-4'-méthanesulfonylbenzophénone**

Formule (IV) : A = phényl, $Y_1$ = 3-F, $Y_2$ = H

Préparé selon le mode opératoire de l'exemple 2.

Cristaux de point de fusion 106°C.

**Exemple 11 : Acide 3-éthoxycarbonyl-4-(3-fluorophényl)-4-(4-méthane sulfonylphényl)-3-buténoïque**

Formule (X) : A = phényl, $Y_1$ = 3-F, $Y_2$ = H

A une solution de 15.7 g (0.140 mole) de t-butylate de potassium dans 100 ml de t-butanol sont ajoutés par portion 35.5 g (0.1275 mole) de 3-fluoro-4'-méthanesulfonylbenzophénone préparée à l'exemple 10. Le mélange est agité et 32 ml (0.191 mole) de succinate d'éthyle sont ajoutés goutte à goutte à un débit rapide. Le mélange est ensuite chauffé au reflux pendant 30 minutes, refroidi et additionné d'eau et d'acide chlorhydrique 1N afin d'obtenir un pH égal à 1 puis extrait avec du t-butyl-méthyl éther. La phase organique est traitée par une solution de soude à 2 % et le mélange est décanté. La phase aqueuse est acidifiée par l'acide chlorhydrique 1N puis extraite avec du t-butyl-méthyl éther. La phase organique est séchée sur sulfate de magnésium et évaporée sous vide pour donner 39.2 g d'acide 3-éthoxycarbonyl-4-(3-fluorophényl)-4-(4-méthanesulfonyl phényl)-3-buténoïque sous forme d'une huile épaisse utilisée telle quelle pour la suite.

**Exemple 12 : 3-(3-fluorophényl)-3-(4-méthanesulfonylphényl)-2-(2-hydroxy éthyl)-2-propénoate d'éthyle**

Formule (XI) : A = phényl, $Y_1$ = 3-F, $Y_2$ = H

A une solution de 31.5 g (0.0775 mole) d'acide 3-éthoxycarbonyl-4-(3-fluorophényl)-4-(4-méthanesulfonylphényl)-3-buténoïque, préparé à l'exemple 11, dans 90 ml de tétrahydrofurane anhydre sont ajoutés goutte à goutte 15.5 ml (0.155 mole) de complexe borane / sulfure de méthyle. Le mélange est agité à température ambiante pendant 8 heures et 23.5 ml de méthanol sont ajoutés goutte à goutte. Le mélange est évaporé à sec sous vide et le résidu est repris à l'acétate d'éthyle, puis traité par une solution aqueuse de 7.6 g de carbonate de potassium. La phase organique est décantée puis séchée sur sulfate de magnésium et évaporée à sec sous vide pour donner 29.3 g de 3-(3-fluorophényl)-3-(4-méthane sulfonylphényl)-2-(2-hydroxyéthyl)-2-propénoate d'éthyle sous forme d'une huile visqueuse utilisée telle quelle pour la suite.

**Exemple 13 : (Z)-3-[1-(3-fluorophényl)-1-(4-méthanesulfonylphényl) méthylidène]-dihydro-furan-2-one**

Isomère (Z) : Formule (I) : A = B = phényl, $Y_1$ = 3-F, $X_2$ = $Y_2$ = H,

$X_1$ = 4-$SO_2CH_3$, $R_1R_2$ = O, $R_3$ = $R_4$ = H

A une solution de 29.3 g de 3-(3-fluorophényl)-3-(4-méthanesulfonyl phényl)-2-(2-hydroxyéthyl)-2-propénoate d'éthyle préparé à l'exemple 12, dans 50 ml d'éthanol, sont ajoutés 3.3 g de soude en solution dans 10 ml d'eau et le mélange est chauffé au reflux pendant 2 heures. Après évaporation à sec, le résidu est repris à l'eau et acidifié par l'acide chlorhydrique 1N puis extrait au dichlorométhane. La phase organique est séchée sur sulfate de magnésium et évaporée sous vide pour donner un résidu huileux. L'huile est solubilisée dans 150 ml de toluène et 10 mg d'acide paratoluène sulfonique sont ajoutés. Le mélange est chauffé au reflux et l'eau formée est éliminée par un Dean Stark. Après refroidissement le mélange est lavé à l'eau, la phase organique est séchée sur sulfate de magnésium, évaporée sous vide et le résidu est chromatographié sur silice dans le t-butyl-méthyl éther pour donner 4 g de (Z) -3-[1-(3-fluorophényl)-1-(4-méthanesulfonylphényl) méthylidène]-dihydro-furan-2-one (2ème produit élué) sous forme de cristaux de point de fusion 153-154°C.

**Exemple 14 : 3,4-dichloro-4'-méthylthiobenzophénone**

Formule (III) : A = phényl, $Y_1$ = 3-Cl, $Y_2$ = 4-Cl

Préparé selon le mode opératoire de l'exemple 1.
Cristaux de point de fusion 100°C.

**Exemple 15 : 3,4-dichloro-4'-méthanesulfonylbenzophénone**

Formule (IV) : A = phényl, $Y_1$ = 3-Cl, $Y_2$ = 4-Cl

Préparé selon le mode opératoire de l'exemple 2.
Cristaux de point de fusion 158°C.

**Exemple 16 : Acide 3-éthoxycarbonyl-4-(3,4-dichlorophényl)-4-(4-méthane sulfonylphényl)-3-buténoïque**

Formule (X) : A = phényl, $Y_1$ = 3-Cl, $Y_2$ = 4-Cl

Préparé selon le mode opératoire de l'exemple 11.
Huile utilisée telle quelle pour la suite.

**Exemple 17: 3-(3,4-dichlorophényl)-3-(4-méthanesulfonylphényl)-2-(2-hydroxyéthyl)-2-propénoate d'éthyle.**

Formule (XI) : A = phényl, $Y_1$ = 3-Cl, $Y_2$ = 4-Cl

Préparé selon le mode opératoire de l'exemple 12.
Huile utilisée telle quelle pour la suite.

**Exemple 18 : (Z)-3-[1-(3,4-dichlorophényl)-1-(4-méthanesulfonylphényl) méthylidènel-dihydro-furan-2-one**

I

Isomère (Z) : Formule (I) : A = B = phényl, $Y_1$=3-Cl, $Y_2$ = 4-Cl,

$X_2$ = H, $X_1$ = 4-$SO_2CH_3$, $R_1R_2$ = O, $R_3$ = $R_4$ = H

Préparé selon le mode opératoire de l'exemple 13. Chromatographié dans un mélange dichlorométhane / acétone (99/1), premier produit élué.
Cristaux de point de fusion 195-197°C.
Lors de la chromatographie, l'isolation du deuxième produit élué permet d'obtenir l'isomère (E)-3-[1-(3,4-dichlorophényl)-1-(4-méthanesulfonyl phényl)méthylidène]-dihydro-furan-2-one sous forme de cristaux de point de fusion 163-164°C.

**Exemple 19 : 3-chloro-4'-méthylthiobenzophénone**

Formule (III) : A = phényl, $Y_1$ = 3-Cl, $Y_2$ = H

Préparé selon le mode opératoire de l'exemple 1.

Cristaux de point de fusion 70°C.

**Exemple 20 : 3-chloro-4'-méthanesulfonylbenzophénone**

Formule (IV) : A = phényl, $Y_1$ = 3-Cl, $Y_2$ = H

Préparé selon le mode opératoire de l'exemple 2.

Cristaux de point de fusion 140°C.

**Exemple 21 : Acide 3-éthoxycarbonyl-4-(3-chlorophényl)-4-(4-méthane sulfonylphényl)-3-buténoïque**

Formule (X) : A = phényl, $Y_1$ = 3-Cl, $Y_2$ = H

Préparé selon le mode opératoire de l'exemple 11.

Huile utilisée telle quelle pour la suite.

**Exemple 22 : 3-(3-chlorophényl)-3-(4-méthanesulfonylphényl)-2-(2-hydroxy éthyl)-2-propénoate d'éthyle**

Formule (XI) : A = phényl, $Y_1$ = 3-Cl, $Y_2$ = H

Préparé selon le mode opératoire de l'exemple 12.

Huile utilisée telle quelle pour la suite.

**Exemple 23 : (Z)-3-[1-(3-chlorophényl)-1-(4-méthanesulfonylphényl) méthylidène]-dihydro-furan-2-one**

Isomère (Z) : Formule (I) : A = B = phényl, $Y_1$ = 3-Cl,

$X_2 = Y_2$ = H, $X_1$ = 4-$SO_2CH_3$, $R_1R_2$ = O, $R_3 = R_4$ = H

Préparé selon le mode opératoire de l'exemple 13. Chromatographié dans un mélange dichlorométhane/acétone (99/1), premier produit élué.

Cristaux de point de fusion 147-149°C.

L'isolation du deuxième produit élué lors de la chromatographie conduit au composé (E) -3-[1-(3-chlorophényl)-1-(4-méthanesulfonylphényl) méthylidène]-dihydro-furan-2-one sous forme de cristaux de point de fusion 170-172°C.

**Exemple 24 : 4-méthylthiobenzophénone**

Formule (III) : A = phényl, $Y_1 = Y_2 = H$

Préparé selon le mode opératoire de l'exemple 1.
Cristaux de point de fusion 84°C.

**Exemple 25 : 4-méthanesulfonylbenzophénone**

Formule (IV) : A = phényl, $Y_1 = Y_2 = H$

Préparé selon le mode opératoire de l'exemple 2.
Cristaux de point de fusion 150°C.

**Exemple 26 : Acide 3-éthoxycarbonyl-4-phényl-4-(4-méthanesulfonyl phényl)-3-buténoïque**

Formule (X) : A = phényl, $Y_1 = Y_2 = H$

Préparé selon le mode opératoire de l'exemple 11.
Huile utilisée telle quelle pour la suite.

**Exemple 27 : 3-phényl-3-(4-méthanesulfonylphényl)-2-(2-hydroxyéthyl)-2-propénoate d'éthyle**

Formule (XI) : A = phényl, $Y_1 = Y_2 = H$

Préparé selon le mode opératoire de l'exemple 12.
Huile utilisée telle quelle pour la suite.

**Exemple 28 : (E)-3-[1-phényl-1-(4-méthanesulfonylphényl)méthylidène]-dihydro-furan-2-one**

Isomère (E) : Formule (I) : A = B = phényl, $Y_1 = Y_2 = H$, $X_2 = H$,

$X_1 = 4\text{-}SO_2CH_3$, $R_1R_2 = O$, $R_3 = R_4 = H$

Préparé selon le mode opératoire de l'exemple 13.
Chromatographié sur gel de silice dans un mélange dichlorométhane / acétone (99/1), premier produit élué.
Cristaux de point de fusion 135-137°C.
L'isolation du 2ème produit élué permet d'obtenir l'isomère (Z)- 3-[1-phényl-1-(4-méthanesulfonylphényl)méthylidène]-dihydro-furan-2-one sous forme de cristaux de point de fusion 206-208°C.

**Exemple 29 : 5,5-diméthyl-3-[1-(4-fluorophényl)-1-hydroxy-1-(4-méthylthio phényl)méthyl]-dihydro-furan-2-one**

Formule (VI') : A = phényl, $Y_1$ = 4-F, $Y_2$ = H, $R_3$ = $R_4$ = $CH_3$

A une suspension de 2.4 g de magnésium en copeaux dans l'éther éthylique anhydre sont ajoutés goutte à goutte 7.5 ml de bromoéthane. A la fin de l'addition, le mélange est refroidi à 0°C et 10.5 ml de N,N-diéthylamine sont ajoutés goutte à goutte. Le milieu réactionnel est agité 1 heure à température ambiante puis chauffé 15 minutes au reflux et refroidi par un bain de glace et de chlorure de sodium. Une solution de 12.3 g de 4-fluoro-4'-méthylthio benzophénone, préparée à l'exemple 1, et de 5.7 g de 4,4-diméthylbutyrolactone dans 50 ml de tétrahydrofurane anhydre est ajoutée goutte à goutte en maintenant la température entre 0°C et 5°C. Le mélange est ensuite porté au reflux pendant 2 heures, refroidi et traité par 100 ml d'une solution d'acide sulfurique à 10 %. Après extraction à l'éther éthylique, la phase organique est séchée sur sulfate de magnésium et évaporée sous vide pour donner 4.8 g de 5,5-diméthyl-3-[1-(4-fluorophényl)-1-hydroxy-1-(4-méthylthiophényl)méthyl]-dihydro-furan-2-one sous forme de cristaux de point de fusion 185°C.

**Exemple 30 : 5,5-diméthyl-3-[1-(4-fluorophényl)-1-(4-méthylthiophényl) méthylidène]-dihydro-furan-2-one**

Isomère (E) : Formule (I) : A = B = phényl, $Y_1$ = 4-F, $Y_2$ = $X_2$ = H,

$X_1$ = 4-$SCH_3$, $R_1R_2$ = O, $R_3$ = $R_4$ = $CH_3$

Isomère (Z) : Formule (I) : A = B = phényl, $X_1$ = 4-F, $X_2$ = $Y_2$ = H,

$Y_1$ = 4-$SCH_3$, $R_1R_2$ = O, $R_3$ = $R_4$ = $CH_3$

Préparé selon le mode opératoire de l'exemple 4 à partir du dérivé de l'exemple 29.

Les deux isomères sont séparés par cristallisation fractionnée dans un mélange éther isopropylique / pentane :

(E)-5,5-diméthyl-3-[1-(4-fluorophényl)-1-(4-méthylthiophényl)méthylidène]-dihydro-furan-2-one, point de fusion 98°C.

(Z)-5,5-diméthyl-3-[1-(4-fluorophényl)-1-(4-méthylthiophényl)méthylidène]-dihydro-furan-2-one, point de fusion 160°C.

**Exemple 31 : (E)-5,5-diméthyl-3-[1-(4-fluorophényl)-1-(4-méthanesulfonyl phényl)méthylidène]-dihydro-furan-2-one**

Isomère (E) : Formule I : A = B = phényl, $Y_1$ = 4-F, $X_2$ = $Y_2$ = H,

$X_1$ = 4-$SO_2CH_3$, $R_1R_2$ = O, $R_3$ = $R_4$ = $CH_3$

A une solution de 2 g de (E)-5,5-diméthyl-3-[1-(4-fluorophényl)-1-(4-méthylthiophényl) méthylidène]-dihydro-furan-2-one, préparé à l'exemple 30, dans 15 ml d'acide acétique sont ajoutés 1.9 g de perborate de sodium trihydraté. Le mélange est chauffé 3 heures à 45°C et les cristaux formés sont essorés à chaud et chromatographiés sur gel de silice dans un mélange dichlorométhane / acétone (9/1) comme éluant pour donner 1.2 g de (E) -5,5-diméthyl-3-[1-(4-fluorophényl)-1-(4-méthanesulfonylphényl)méthylidène]-dihydro-furan-2-one sous forme de cristaux de point de fusion 175°C.

**Exemple 32 : 5-éthyl-3-[1-(4-fluorophényl)-1-hydroxy-1-(4-méthylthio phényl) méthyl]-dihydro-furan-2-one**

Formule (VI') : A = phényl, $Y_1$ = 4-F, $Y_2$ = H, $R_3$ = $C_2H_5$, $R_4$ = H

Préparé selon le mode opératoire de l'exemple 29 à partir de la γ-caprolactone.
Cristaux de point de fusion 150°C.

**Exemple 33 : 5-éthyl-3-[1-(4-fluorophényl)-1-(4-méthylthiophényl) méthylidène]-dihydro-furan-2-one**

Isomère (E) : Formule (I) : A = B = phényl, $Y_1$ = 4-F, $X_2$ = $Y_2$ = H,

$X_1$ = 4-$SCH_3$, $R_1R_2$ = O, $R_3$ = $C_2H_5$, $R_4$ = H

Isomère (Z) : Formule (I) : A = B = phényl, $X_1$ = 4-F, $X_2$ = $Y_2$ = H,

$Y_1$ = 4-$SCH_3$, $R_1R_2$ = O, $R_3$ = $C_2H_5$, $R_4$ = H

Préparé selon le mode opératoire de l'exemple 4 à partir du dérivé de l'exemple 32. Le mélange de deux isomères (E) et (Z) est utilisé tel quel pour la suite.

**Exemple 34 : (E)-5-éthyl-3-[1-(4-fluorophényl)-1-(4-méthanesulfonylphényl) méthylidènel-dihydro-furan-2-one**

Isomère (E) : Formule (I) : A = B = phényl, $Y_1$ = 4-F, $X_2$ = $Y_2$ = H,

$X_1$ = 4-$SO_2CH_3$, $R_1R_2$ = O, $R_3$ = $C_2H_5$, $R_4$ = H

Préparé selon le mode opératoire de l'exemple 31. L'isomère (E) -5-éthyl-3-[1-(4-fluorophényl)-1-(4-méthanesulfonylphényl)méthylidène]-dihydrofuran-2-one est purifié par chromatographie sur gel de silice dans un mélange dichlorométhane / acétone (99/1), sous forme de cristaux de point de fusion 134-136°C (premier produit élué).
L'isolation du deuxième produit élué lors de la chromatographie permet d'obtenir le (Z) -5-éthyl-3-[1-(4-fluorophényl)-1-(4-méthanesulfonylphényl) méthylidène]-dihydro-furan-2-one sous forme de cristaux de point de fusion 176-177°C.

**Exemple 35 : 5-méthyl-3-[1-(3-chlorophényl)-1-hydroxy-1-(4-méthylthio phényl)méthyl]-dihydro-furan-2-one**

Formule (VI') : A = phényl, $Y_1$ = 3-Cl, $Y_2$ = H, $R_3$ = $CH_3$,

$R_4$ = H

Préparé selon le mode opératoire de l'exemple 6 à partir de la 3-chloro-4'-méthylthiobenzophénone et de l'α-bromo-γ-valérolactone.
Poudre amorphe utilisée telle quelle pour la suite;

**Exemple 36 : 5-méthyl-3-[1-(3-chlorophényl)-1-(4-méthylthiophényl) méthylidène]-dihydro-furan-2-one**

Isomère (Z) : Formule (I) : A = B = phényl, $Y_1$ = 3-Cl,

$X_2 = Y_2$ = H, $X_1$ = 4-$SCH_3$, $R_1R_2$ = O, $R_3$ = $CH_3$, $R_4$ = H

Isomère (E) : Formule (I) : A = B = phényl, $X_1$ = 3-Cl,

$X_2 = Y_2$ = H, $Y_1$ = 4-$SCH_3$, $R_1R_2$ = O, $R_3$ = $CH_3$, $R_4$ = H

Préparé selon le mode opératoire de l'exemple 4 à partir du dérivé de l'exemple 35. Le mélange des deux isomères est utilisé tel quel pour la suite sous forme d'huile.

**Exemple 37 : (Z)-5-méthyl-3-[1-(3-chlorophényl)-1-(4-méthanesulfonyl phényl)méthyl]-dihydro-furan-2-one**

Isomère (Z) : Formule (I) : A = B = phényl, $Y_1$ = 3-Cl,

$X_2 = Y_2$ = H, $X_1$ = 4-$SO_2CH_3$, $R_1R_2$ = O, $R_3$ = $CH_3$, $R_4$ = H

Préparé selon le mode opératoire de l'exemple 31. L'isomère (Z) -5-méthyl-3-[1-(3-chlorophényl)-1-(4-méthanesulfonylphényl)méthyl]-dihydrofuran-2-one est purifié par chromatographie sur gel de silice dans un mélange dichlorométhane / acétone (99/1)comme éluant sous forme d'une poudre amorphe (premier produit élué).

L'isolation du deuxième produit élué permet d'obtenir l'isomère (E) -5-méthyl-3-[1-(3-chlorophényl)-1-(4-méthanesulfonylphényl)méthyl]-dihydrofuran-2-one sous forme de cristaux de point de fusion 164-165°C.

**Exemple 38 : 2-chloro-5-(4-méthylthiobenzoyl)pyridine**

Formule (III) : A = 3-pyridyl, $Y_1$ = 6-Cl, $Y_2$ = H

Préparé selon le mode opératoire de l'exemple 1.

Cristaux de point de fusion 145°C.

**Exemple 39 : 2-chloro-5-(4-méthanesulfonylbenzoyl)pyridine**

Formule (IV) : A = 3-pyridyl, $Y_1$ = 6-Cl, $Y_2$ = H

=

Une solution de 34.6 g de 2-chloro-5-(4-méthylthiobenzoyl)pyridine préparée à l'exemple 38 et de 42 g de perborate de sodium trihydraté dans 250 ml d'acide acétique est chauffée 4 heures à 45°C. Les cristaux formés sont essorés à chaud, lavés à l'eau et séchés pour donner 32.6 g de 2-chloro-5-(4-méthane sulfonyl benzoyl)pyridine sous forme de cristaux de point de fusion 170°C.

**Exemple 40 : Acide 4-(6-chloropyridin-3-yl)-3-éthoxycarbonyl-4-(4-méthanesulfonylphényl)-3-buténoïque**

Formule (X) : A = 3-pyridyl, $Y_1$ = 6-Cl, $Y_2$= H

Préparé selon le mode opératoire de l'exemple 11 à partir du dérivé de l'exemple 39.
Solide amorphe utilisé tel quel pour la suite.

**Exemple 41 : 3-(6-chloropyridin-3-yl)-2-(2-hydroxyéthyl)-3-(4-méthane sulfonylphényl)-2-propénoate d'éthyle**

Formule (XI) : A = 3-pyridyl, $Y_1$ = 6-Cl, $Y_2$ = H

Préparé selon le mode opératoire de l'exemple 12, à partir du dérivé de l'exemple 40.
Solide amorphe utilisé tel quel pour la suite.

**Exemple 42 : (Z)-3-[1-(6-chloropyridin-3-yl)-1-(4-méthanesulfonylphényl) méthylidène)-dihydro-furan-2-one**

Isomère (Z) : Formule (I) : A = 3-pyridyl, B = phényl

$Y_1$ = 6-Cl, $X_2$ = $Y_2$ = H, $X_1$ = 4-$SO_2CH_3$, $R_1R_2$ = O, $R_3$ = $R_4$ = H

Préparé selon le mode opératoire de l'exemple 13, à partir du dérivé de l'exemple 41.
L'isomère (Z) est obtenu par chromatographie dans un mélange dichlorométhane / acétone (5/1) puis cristallisation dans un mélange acétone / éther éthylique sous forme de cristaux de point de fusion 172-174°C.
L'isomère (E) -3-[1-(6-chloropyridin-3-yl)-1-(4-méthanesulfonylphényl) méthylidène]-dihydro-furan-2-one est obtenu pur par cristallisation dans l'acétone, avant chromatographie du mélange brut des deux isomères, sous forme de cristaux de point de fusion 198-199°C.

**Exemple 43 : Acide 3-éthoxycarbonyl-4-(4-fluorophényl)-4-(4-méthane sulfonylphényl)-3-buténoïque**

Formule (X) : A = phényl, $Y_1$ = 4-F, $Y_2$ = H

Préparé selon le mode opératoire de l'exemple 11 à partir de la 4-fluoro-4'-méthanesulfonyl benzophénone préparée à l'exemple 2.
Huile utilisée telle quelle pour la suite.

**Exemple 44 : 4-[1-(4-fluorophényl)-1-(4-méthanesulfonylphényl)méthylidène] -dihydro-furan-2-one**

Formule (I) : A = B = phényl, $X_1$ = 4-$SO_2CH_3$, $X_2$ = $Y_2$ = H,

$Y_1$ = 4-F, $R_1R_2$=H, $R_3R_4$ = O

A une solution d'acide 3-éthoxycarbonyl-4-(4-fluorophényl)-4-(4-méthanesulfonylphényl)-3-buténoïque, préparé à l'exemple 43, dans 500 ml d'éthanol sont ajoutés 13.6 g de chlorure de calcium anhydre en poudre. Le mélange

est agité à température ambiante et 7.5 g de borohydrure de sodium en solution dans un mélange composé de 1.5 g de potasse, 10 ml d'eau et 10 ml d'éthanol, sont ajoutés goutte à goutte en refroidissant par un bain de glace. Après 4 heures à température ambiante, le milieu réactionnel est refroidi à 0°C et une solution d'acide chlorhydrique 6N est ajoutée goutte à goutte. Après extraction au dichlorométhane, la phase organique est séchée sur sulfate de magnésium, évaporée sous vide et le résidu cristallisé dans l'éther isopropylique. Les cristaux obtenus sont recristallisés dans le méthanol pour donner 5 g de 4-[1-(4-fluorophényl)-1-(4-méthanesulfonylphényl)méthylidène]-dihydro-furan-2-one sous forme de mélange (50/50) des deux isomères (E) et (Z), cristaux de point de fusion 160-164°C.

**Exemple 45 : 3-méthyl-4'-méthylthiobenzophénone**

Formule (III) : A = phényl, $Y_1$ = 3-$CH_3$, $Y_2$ = H

Préparé selon le mode opératoire de l'exemple 1.

Cristaux de point de fusion 46-47°C.

**Exemple 46 : 3-méthyl-4'-méthanesulfonylbenzophénone**

Formule (IV) : A = phényl, $Y_1$ = 3-$CH_3$, $Y_2$ = H

Préparé selon le mode opératoire de l'exemple 2.

Cristaux de point de fusion 150°C.

**Exemple 47 : Acide 3-éthoxycarbonyl-4-(3-méthylphényl)-4-(4-méthane sulfonylphényl)-3-buténoïque**

Formule (X) : A = phényl, $Y_1$ = 3-$CH_3$, $Y_2$ = H

Préparé selon le mode opératoire de l'exemple 11, à partir du dérivé de l'exemple 46.

Huile utilisée telle quelle pour la suite.

**Exemple 48 : 3-(3-méthylphényl)-3-(4-méthanesulfonylphényl)-2-(2-hydroxy éthyl)-2-propénoate d'éthyle**

Formule (XI) : A = phényl, $Y_1$ = 3-$CH_3$, $Y_2$ = H

Préparé selon le mode opératoire de l'exemple 12, à partir du dérivé de l'exemple 47.

Huile utilisée telle quelle pour la suite.

**Exemple 49 : (Z)-3-[1-(3-méthylphényl)-1-(4-méthanesulfonylphényl) méthylidène]-dihydro-furan-2-one**

Isomère (Z) : Formule (I) : A = B = phényl, $Y_1$ = 3-$CH_3$,

$X_2 = Y_2 = H, X_1 = 4\text{-}SO_2CH_3, R_1R_2 = O, R_3 = R_4 = H$

Préparé selon le mode opératoire de l'exemple 13, à partir du dérivé de l'exemple 48.

Purifié par chromatographie sur gel de silice dans un mélange dichlorométhane / acétone (99/1), premier produit élué, sous forme de cristaux de point de fusion 166°C.

**Exemple 50 : 3,4-difluoro-4'-méthylthiobenzophénone**

Formule (III) : A = phényl, $Y_1$ = 3-F, $Y_2$ = 4-F

Préparé selon le mode opératoire de l'exemple 1.
Cristaux de point de fusion 96°C.

**Exemple 51 : 3,4-difluoro-4'-méthanesulfonylbenzophénone**

Formule (IV) : A = phényl, $Y_1$ = 3-F, $Y_2$ = 4-F

Préparé selon le mode opératoire de l'exemple 2, à partir du dérivé de l'exemple 50.
Cristaux de point de fusion 120°C.

**Exemple 52 : Acide 3-éthoxycarbonyl-4-(3,4-difluorophényl)-4-(méthane sulfonylphényl)-3-buténoïque**

Formule (X) : A = phényl, $Y_1$ = 3-F, $Y_2$ = 4-F

Préparé selon le mode opératoire de l'exemple 11, à partir du dérivé de l'exemple 51.
Huile utilisée telle quelle pour la suite.

**Exemple 53 : 3-(3,4-difluorophényl)-3-(4-méthanesulfonylphényl)-2-(2-hydroxyéthyl)-2-propénoate d'éthyle**

Formule (XI) : A = phényl, $Y_1$ = 3-F, $Y_2$ = 4-F

Préparé selon le mode opératoire de l'exemple 12, à partir du dérivé de l'exemple 52.
Huile utilisée telle quelle pour la suite.

**Exemple 54 : (Z)-3-[1-(3,4-difluorophényl)-1-(4-méthanesulfonylphényl) méthylidène]-dihydro-furan-2-one**

Isomère (Z) : Formule (I) : A = B = phényl, $Y_1$ = 3-F,

$Y_2$ = 4-F, $X_1$ = 4-$SO_2CH_3$, $X_2$ = H, $R_1R_2$ = O, $R_3$ = $R_4$ = H

Préparé selon le mode opératoire de l'exemple 13, à partir du dérivé de l'exemple 53.
Purifié par chromatographie sur gel de silice dans un mélange dichlorométhane / acétone (99/1), premier produit élué, cristaux de point de fusion 148°C.

**Exemple 55 : 4-benzylthiobenzonitrile**

Un mélange de 37.2 g de benzylmercaptan, 36.3 g de 4-fluoro benzonitrile et 42 g de carbonate de potassium dans 700 ml de 2-butanone est porté au reflux pendant 7 heures. Le solvant est évaporé sous vide, le résidu est repris à l'eau et à l'éther de pétrole. Les cristaux formés sont essorés, lavés à l'eau et à l'éther de pétrole pour donner 46 g de 4-benzylthiobenzonitrile sous forme de cristaux de point de fusion 85°C.

**Exemple 56 : 4-benzylthio-4'-fluorobenzophénone**

A une suspension de 9.6 g de magnésium en copeaux dans 20 ml d'éther éthylique anhydre est ajoutée goutte à goutte une solution de 44 ml de 4-bromo-1-fluorobenzène dans 300 ml d'éther éthylique anhydre. Après la fin de l'addition, le mélange est agité quelques minutes à température ambiante et une solution de 46 g de 4-benzylthiobenzonitrile, préparé à l'exemple 55, dans 400 ml de tétrahydrofurane anhydre est ajoutée goutte à goutte. L'éther éthylique est distillé et le mélange est porté au reflux pendant 3 heures puis refroidi par de la glace. Une solution d'acide chlorhydrique 6N (400 ml) est ajoutée goutte à goutte et le mélange est porté au reflux pendant 6 heures. Après addition d'eau et de dichlorométhane, la phase organique est décantée et séchée sur sulfate de magnésium, puis évaporée sous vide. Le résidu cristallise dans l'éther isopropylique pour donner 48 g de 4-benzylthio-4'-fluorobenzophénone sous forme de cristaux de point de fusion 96°C.

**Exemple 57 : 4-t-butylaminosulfonyl-4'-fluorobenzophénone**

Dans une solution de 43 g de 4-benzylthio-4'-fluorobenzophénone préparé à l'exemple 56, dans 300 ml d'acide acétique refroidi à 0°C, est mis à barbotter du chlore jusqu'à saturation (36 g). Le mélange est ensuite agité 2 heures à température ambiante puis versé sur de l'eau glacée et extrait au dichlorométhane. La phase organique est lavée à l'eau, séchée sur sulfate de magnésium et évaporée sous vide pour donner 47 g d'une huile qui est mise en solution dans 100 ml de 1,2-dichloroéthane. Cette solution est ajoutée goutte à goutte à une solution de 50 ml de t-butylamine dans 300 ml de 1,2-dichloroéthane. Le mélange est chauffé une heure à 80°C, refroidi et lavé à l'eau puis à l'acide chlorhydrique dilué. La phase organique est séchée sur sulfate de magnésium et évaporée sous vide. Le résidu cristallise dans l'éther éthylique pour donner 25 g de 4-t-butylaminosulfonyl-4'-fluorobenzophénone sous forme de cristaux de point de fusion 160°C.

**Exemple 58 : Acide 3-éthoxycarbonyl-4-(4-t-butylaminosulfonylphényl)-4-(4-fluorophényl)-3-buténoïque**

Formule (XII) : A = phényl, $Y_1$ = 4-F, $Y_2$ = H

Préparé selon le mode opératoire de l'exemple 11, à partir du dérivé de l'exemple 57 en utilisant 2 équivalents de t-butylate de potassium.

Poudre amorphe utilisée telle quelle pour la suite.

**Exemple 59 : 3-(4-t-butylaminosulfonylphényl)-3-(4-fluorophényl)-2-(2-hydroxyéthyl)-2-propénoate d'éthyle**

Préparé selon le mode opératoire de l'exemple 12, à partir du dérivé de l'exemple 58.

L'huile obtenue est reprise à l'éther éthylique et les cristaux formés sont séchés pour donner l'isomère (Z)-3-(4-t-butylaminosulfonylphényl)-3-(4-fluorophényl)-2- (2-hydroxyéthyl)-2-propénoate d'éthyle pur sous forme de cristaux de point de fusion 152°C. Le filtrat est évaporé sous vide pour donner un résidu huileux correspondant à l'isomère (E)-3-(4-t-butylaminosulfonylphényl)-3-(4-fluorophényl)-2-(2-hydroxyéthyl)-2-propénoate d'éthyle utilisé tel quel pour la suite.

**Exemple 60 : (E)-4-[(4-fluorophényl)-(2-oxo-dihydro-furan-3-ylidène)méthyl] benzènesulfonamide**

Isomère (E) : Formule (I) : A = B= phényl, $Y_1$ = 4-F, $Y_2$ = $X_2$ = H,

$$X_1 = 4\text{-}SO_2NH_2,\ R_1R_2 = O,\ R_3 = R_4 = H$$

Le (E)-3-(4-t-butylaminosulfonylphényl)-3-(4-fluorophényl)-2-(2-hydroxyéthyl)-2-propénoate d'éthyle (10 g), préparé à l'exemple 59, est mis en solution dans 20 ml d'éthanol et 2 g de soude en solution dans 10 ml d'eau sont ajoutés. Le mélange est chauffé au reflux pendant 2 h. Après évaporation à sec, le résidu est repris par de l'eau et acidifié par l'acide chlorhydrique 1N puis extrait au dichlorométhane. La phase organique est séchée sur sulfate de magnésium puis évaporée sous vide.Le résidu est ajouté par portions à 500 ml d'acide sulfurique concentré. Le mélange est agité 15 minutes à température ambiante puis versé sur de l'eau glacée, les cristaux formés sont essorés et lavés à l'éther éthylique puis repris dans 100 ml d'acétone tiède. A la solution obtenue sont ajoutés 50 ml d'éther éthylique et les cristaux formés sont essorés et séchés pour donner 6 g de (E)-4-[(4-fluorophényl)-(2-oxo-dihydro-furan-3-ylidène)méthyl]benzène sulfonamide sous forme de cristaux de point de fusion 202°C.

**Exemple 61 : (Z)-4-[(4-fluorophényl)-(2-oxo-dihydro-furan-3-ylidène)méthyl] benzènesulfonamide**

Isomère (Z) : Formule (I) : A = B= phényl, $X_1$ = 4-F, $X_2 = Y_2$ = H,

$$Y_1 = 4\text{-}SO_2NH_2,\ R_1R_2 = O,\ R_3 = R_4 = H$$

Préparé selon le mode opératoire de l'exemple 60, à partir de l'isomère (Z) préparé à l'exemple 59.
Cristaux de point de fusion 222°C.

**Exemple 62 : 4-chloro-4'-méthanesulfonylbenzophénone**

Formule (IV) : A = phényl, $Y_1$ = 4-Cl, $Y_2$ = H

Préparé selon le mode opératoire de l'exemple 39 à partir du dérivé de l'exemple 5.
Cristaux de point de fusion 176°C.

**Exemple 63 : Acide (Z)-3-étboxycarbonyl-4-(4-chlorophényl)-4-(4-méthanesulfonyl)-3-buténoïque**

Formule (X) : A = phényl, $Y_1$ = 4-Cl, $Y_2$ = H

A une suspension de 20 g de 4-chloro-4'-méthanesulfonylbenzophénone, préparée à l'exemple 62, dans 100 ml de t-butanol sont ajoutés en une fois 7.2 g de t-butylate de sodium à température ambiante. La suspension est chauffée à 40°C et une solution de 16.9 ml de succinate d'éthyle dans 20 ml de t-butanol est ajoutée en 10 minutes. La température est maintenue à *55°C* pendant 30 minutes puis ramenée à 35-40°C ; 140 ml d'eau froide sont ajoutés et le mélange est agité pendant 30 minutes. La solution est filtrée et les cristaux sont lavés à l'eau. La phase hydroalcoolique est lavée deux fois avec 75 ml de toluène puis acidifiée par de l'acide chlorhydrique concentré. Les cristaux sont essorés, lavés à l'eau et séchés sous vide pour donner 20.2 g d'acide (Z)-3-éthoxycarbonyl-4-(4-chloro phényl)-4-(4-méthanesulfonyl)-3-buténoïque, pureté HPLC : 81.6 %, 12.1 % d'isomère (E). La recristallisation dans le 2-propanol permet d'obtenir 14 g d'acide (Z)-3-éthoxycarbonyl-4-(4-chlorophényl)-4-(4-méthanesulfonyl)-3-buténoïque de pureté HPLC : 96.1 % contenant 0.7 % d'isomère (E), sous forme de cristaux de point de fusion 183°C.

**Exemple 64 : (Z)-3-(4-chlorophényl)-3-(4-méthanesulfonylphényl)-2-(2-hydroxyéthyl)-2-propénoate d'éthyle**

Formule (XI) : A = phényl, $Y_1$ = 4-Cl, $Y_2$ = H

A une solution de 350 g d'acide (Z)-3-éthoxycarbonyl-4-(4-chloro phényl)-4-(4-méthanesulfonylphényl)-3-buténoïque, préparé à l'exemple 63, dans 1.5 1 de tétrahydrofurane sont ajoutés goutte à goutte et sous bonne agitation 120 ml de complexe borane / sulfure de méthyle. Après la fin de l'addition, la solution est agitée pendant 2.5 heures à température ambiante. L'excès de borane est hydrolysé par 100 ml de méthanol et 100 ml d'eau. Après évaporation sous vide du solvant, le résidu est organisé dans l'eau, filtré, lavé à l'eau pour donner le (Z)-3-(4-chlorophényl)-3-(4-méthanesulfonylphényl)-2-(2-hydroxyéthyl)-2-propénoate d'éthyle sous forme de cristaux humides, utilisés tels quels pour l'étape suivante. Par séchage de ces cristaux et recristallisation dans le 2-propanol on obtient le (Z) )-3-(4-chlorophényl)-3-(4-méthanesulfonylphényl)-2-(2-hydroxyéthyl)-2-propénoate d'éthyle sous forme de cristaux de pureté HPLC 98.3 % et de point de fusion 128°C.

**Exemple 65 : (Z)-3-[1-(4-chlorophényl)-1-(4-méthanesulfonylphényl) méthylidène]-dihydro-furan-2-one**

Formule (I) : A = B = phényl, $Y_1$ = 4-Cl, $X_2$ = $Y_2$ = H,

$X_1$ = 4-$SO_2CH_3$, $R_1R_2$ = O, $R_3$ = $R_4$ = H

Le produit humide de l'exemple 64 est chauffé au reflux dans 1.5 l de toluène en présence de 1 g d'acide 4-toluènesulfonique. L'eau et l'éthanol sont éliminés à l'aide d'un Dean Starck puis environ 1l de toluène est évaporé. Après retour à température ambiante les cristaux formés sont essorés et lavés avec une quantité minimale de 2-butanone, puis séchés pour donner 261.5 g de (Z)-3-[1-(4-chlorophényl)-1-(4-méthanesulfonylphényl) méthylidène]-dihydro-furan-2-one sous forme de cristaux de point de fusion 188-190°C.

**Exemple 66 : 3,5-dichloro-4'-méthylthiobenzophénone**

Formule (III) : A = phényl, $Y_1$ = 3-Cl, $Y_2$ = 5-Cl

Préparé selon le mode opératoire de l'exemple 1.
Cristaux de point de fusion 108°C.

**Exemple 67 : 3,5-dichloro-4'-méthanesulfonylbenzophénone**

Formule (IV) : A = phényl, $Y_1$ = 3-Cl, $Y_2$ = 5-Cl

Préparé selon le mode opératoire de l'exemple 39.
Cristaux de point de fusion 200°C.

**Exemple 68 : Acide (Z)-3-éthoxycarbonyl-4-(3,5-dichlorophényl)-4-(4-méthanesulfonylphényl)-3-buténoïque**

Isomère (Z) : Formule (X) : A = phényl, $Y_1$ = 3-Cl, $Y_2$ = 5-Cl

Préparé selon le mode opératoire de l'exemple 11.
Cristaux de point de fusion 180°C.

**Exemple 69 : (Z)-3-(3,5-dichlorophényl)-3-(4-méthanesulfonylphényl) -2-(2-hydroxyéthyl)-2-propénoate d'éthyle**

Isomère (Z) : Formule (XI) : A = phényl, $Y_1$ = 3-Cl, $Y_2$ = 5-Cl

Préparé selon le mode opératoire de l'exemple 12.
Huile utilisée telle quelle pour la suite.

**Exemple 70 : (Z)-3-[1-(3,5-dichlorophényl)-1-(4-méthanesulfonylphényl) méthylidène]-dihydro-furan-2-one**

Isomère (Z) : Formule (I) :A = B = phényl, $Y_1$ = 3-Cl, $Y_2$ = 5-Cl,

$X_1$ = 4-$SO_2CH_3$, $X_2$=H,$R_1R_2$=O,$R_3$=$R_4$=H

Préparé selon le mode opératoire de l'exemple 65.
Cristaux de point de fusion 114°C.

**Exemple 71 : 2,4-difluoro-4'-méthylthiobenzophénone**

Formule (III) : A = phényl, $Y_1$ = 2-F, $Y_2$ = 4-F

Préparé selon le mode opératoire de l'exemple 1.
Cristaux de point de fusion 98°C.

**Exemple 72 : 2,4-difluoro-4'-méthanesulfonylbenzophénone**

Formule (IV) : A = phényl, $Y_1$ = 2-F, $Y_2$ = 4-F

Préparé selon le mode opératoire de l'exemple 39.
Cristaux de point de fusion 160°C.

**Exemple 73 : Acide 3-éthoxycarbonyl-4-(2,4-difluorophényl)-4-(4-méthanesulfonylphényl)-3-buténoïque**

Formule (X) : A = phényl, $Y_1$ = 2-F, $Y_2$ = 4-F

Préparé selon le mode opératoire de l'exemple 11.
Huile utilisée telle quelle pour la suite.

**Exemple 74 : 3-(2,4-difluorophényl)-3-(4-méthanesulfonylphényl) -2-(2-hydroxyéthyl)-2-propénoate d'éthyle**

Formule (XI) : A = phényl, $Y_1$ = 2-F, $Y_2$ = 4-F

Préparé selon le mode opératoire de l'exemple 12.
Huile utilisée telle quelle pour la suite.

**Exemple 75 : (Z)-3-[1-(2,4-difluorophényl)-1-(4-méthanesulfonylphényl) méthylidène]-dihydro-furan-2-one**

Isomère (Z) : Formule (I) :A = B = phényl, $Y_1$ = 2-F, $Y_2$ = 4-F,

$X_1$ = 4-$SO_2CH_3$, $X_2$ = H, $R_1R_2$ = O, $R_3$ = $R_4$ = H

Préparé selon le mode opératoire de l'exemple 65, purifié par chromatographie dans un mélange dichlorométhane / acétone (10/0.3).
Cristaux de point de fusion 140°C.

**Exemple 76 : 3,5-difluoro-4'-méthylthiobenzophénone**

Formule (III) : A = phényl, $Y_1$ = 3-F, $Y_2$ = 5-F

Préparé selon le mode opératoire de l'exemple 1.
Cristaux de point de fusion 90°C.

**Exemple 77 : 3,5-difluoro-4'-méthanesulfonylbenzophénone**

Formule (IV) : A = phényl, $Y_1$ = 3-F, $Y_2$ = 5-F

Préparé selon le mode opératoire de l'exemple 39.
Cristaux de point de fusion 152°C.

**Exemple 78 : Acide 3-éthoxycarbonyl-4-(3,5-difluorophényl)-4-(4-méthanesulfonylphényl)-3-buténoïque**

Formule (X) : A = phényl, $Y_1$ = 3-F, $Y_2$ = 5-F

Préparé selon le mode opératoire de l'exemple 11.
Huile utilisée telle quelle pour la suite.

**Exemple 79 : 3-(3,5-difluorophényl)-3-(4-méthanesulfonylphényl) -2-(2-hydroxyéthyl)-2-propénoate d'éthyle**

Formule (XI) : A = phényl, $Y_1$ = 3-F, $Y_2$ = 5-F

Préparé selon le mode opératoire de l'exemple 12.
Huile utilisée telle quelle pour la suite.

**Exemple 80 : (Z)-3-[1-(3,5-difluorophényl)-1-(4-méthanesulfonylphényl) méthylidène]-dihydro-furan-2-one**

Isomère (Z) : Formule (I) :A = B = phényl, $Y_1$ = 3-F, $Y_2$ = 5-F,

$X_1$ = 4-$SO_2CH_3$, $X_2$ = H, $R_1R_2$ = O, $R_3$ = $R_4$ = H

Préparé selon le mode opératoire de l'exemple 65, purifié par chromatographie dans un mélange dichlorométhane/acétone (10/0.3).
Cristaux de point de fusion 162°C.

**Exemple 81 : 4-méthyl-4'-méthylthiobenzophénone**

Formule (III) : A = phényl, $Y_1$ = 4-$CH_3$, $Y_2$ = H

Préparé selon le mode opératoire de l'exemple 1.
Cristaux de point de fusion 96°C.

**Exemple 82 : 4-méthyl-4'-méthanesulfonylbenzophénone**

Formule (IV) : A = phényl, $Y_1$ = 4-$CH_3$, $Y_2$ = H

Préparé selon le mode opératoire de l'exemple 39.
Cristaux de point de fusion 180°C.

**Exemple 83 : Acide 3-éthoxycarbonyl-4-(4-méthylphényl)-4-(4-méthanesulfonylphényl)-3-buténoïque**

Formule (X) : A = phényl, $Y_1$ = 4-$CH_3$, $Y_2$ = H

Préparé selon le mode opératoire de l'exemple 11.
Huile utilisée telle quelle pour la suite.

**Exemple 84 : 3-(4-méthylphényl)-3-(4-méthanesulfonylphényl) -2-(2-hydroxyéthyl)-2-propénoate d'éthyle**

Formule (XI) : A = phényl, $Y_1$ = 4-$CH_3$, $Y_2$ = H

Préparé selon le mode opératoire de l'exemple 12.
Huile utilisée telle quelle pour la suite.

**Exemple 85 : (E)-3-[1-(4-méthylphényl)-1-(4-méthanesulfonylphényl) méthylidène]-dihydro-furan-2-one**

Isomère (E) : Formule (I) :A = B = phényl, $Y_1$ = 4-$CH_3$, $Y_2$ = H,

$X_1$ = 4-$SO_2CH_3$, $X_2$ = H, $R_1R_2$ = O, $R_3$ = $R_4$ = H

Préparé selon le mode opératoire de l'exemple 65, purifié par chromatographie dans un mélange dichlorométhane/acétone (10/0.3).
Cristaux de point de fusion 222°C.

**Exemple 86 : 3-bromo-4'-méthylthiobenzophénone**

Formule (III) : A = phényl, $Y_1$ = 3-Br, $Y_2$ = H

Préparé selon le mode opératoire de l'exemple 1.
Cristaux de point de fusion 90°C.

**Exemple 87 : 3-bromo-4'-méthanesulfonylbenzophénone**

Formule (IV) : A = phényl, $Y_1$ = 3-Br, $Y_2$ = H

Préparé selon le mode opératoire de l'exemple 39.
Cristaux de point de fusion 170°C.

**Exemple 88 : Acide 3-éthoxycarbonyl-4-(3-bromophényl)-4-(4-méthanesulfonylphényl)-3-buténoïque**

Formule (X) : A = phényl, $Y_1$ = 3-Br, $Y_2$ = H

Préparé selon le mode opératoire de l'exemple 11.
Huile utilisée telle quelle pour la suite.

**Exemple 89 : 3-(3-bromophényl)-3-(4-méthanesulfonylphényl) -2-(2-hydroxyéthyl)-2-propénoate d'éthyle**

Formule (XI) : A = phényl, $Y_1$ = 3-Br, $Y_2$ = H

Préparé selon le mode opératoire de l'exemple 12.
Huile utilisée telle quelle pour la suite.

**Exemple 90 : (Z)-3-[1-(3-bromophényl)-1-(4-méthanesulfonylphényl) méthylidènel-dihydro-furan-2-one**

Isomère (Z) : Formule (I) :A = B = phényl, $Y_1$ = 3-Br, $Y_2$ = H,

$X_1$ = 4-$SO_2CH_3$, $X_2$ = H, $R_1R_2$ = O, $R_3$ = $R_4$ = H

Préparé selon le mode opératoire de l'exemple 65, purifié par chromatographie dans un mélange dichlorométhane/acétone (10/0.3).
Cristaux de point de fusion 162°C.

**Exemple 91 : 3-chloro-4-fluoro-4'-méthylthiobenzophénone**

Formule (III) : A = phényl, $Y_1$ = 3-Cl, $Y_2$ = 4-F

Préparé selon le mode opératoire de l'exemple 1.
Cristaux de point de fusion 116°C.

**Exemple 92 : 3-chloro-4-fluoro-4'-méthanesulfonylbenzophénone**

Formule (IV) : A = phényl, $Y_1$ = 3-Cl, $Y_2$ = 4-F

Préparé selon le mode opératoire de l'exemple 39.
Cristaux de point de fusion 146°C.

**Exemple 93 : Acide 3-éthoxycarbonyl-4-(3-chloro-4-fluorophényl)-4-(4-méthanesulfonylphényl)-3-buténoïque**

Formule (X) : A = phényl, $Y_1$ = 3-Cl, $Y_2$ = 4-F

Préparé selon le mode opératoire de l'exemple 11.
Huile utilisée telle quelle pour la suite.

**Exemple 94 : 3-(3-chloro-4-fluorophényl)-3-(4-méthanesulfonylphényl) -2-(2-hydroxyéthyl)-2-propénoated'éthyle**

Formule (XI) : A = phényl, $Y_1$ = 3-Cl, $Y_2$ = 4-F

Préparé selon le mode opératoire de l'exemple 12.
Huile utilisée telle quelle pour la suite.

**Exemple 95 : (Z)-3-[1-(3-chloro-4-fluorophényl)-1-(4-méthanesulfonylphényl) méthylidène]-dihydro-furan-2-one**

Isomère (Z) : Formule (I) :A = B = phényl, $Y_1$ = 3-Cl, $Y_2$ = 4-F,

$X_1$ = 4-$SO_2CH_3$, $X_2$ = H, $R_1R_2$ = O, $R_3$ = $R_4$ = H

Préparé selon le mode opératoire de l'exemple 65, purifié par chromatographie dans un mélange dichlorométhane/acétone (10/0.3).
Cristaux de point de fusion 123°C.

**Exemple 96 : 4-bromo-4'-méthylthiobenzophénone**

Formule (III): A = phényl, $Y_1$ = 4-Br, $Y_2$ = H

Préparé selon le mode opératoire de l'exemple 1.
Cristaux de point de fusion 148°C.

**Exemple 97 : 4-bromo-4'-méthanesulfonylbenzophénone**

Formule (IV) : A = phényl, $Y_1$ = 4-Br, $Y_2$ = H

Préparé selon le mode opératoire de l'exemple 39.
Cristaux de point de fusion 188°C.

**Exemple 98 : Acide 3-éthoxycarbonyl-4-(4-bromophényl)-4-(4-méthanesulfonylphényl)-3-buténoïque**

Formule (X) : A = phényl, $Y_1$ = 4-Br, $Y_2$ = H

Préparé selon le mode opératoire de l'exemple 11.
Huile utilisée telle quelle pour la suite.

**Exemple 99 : 3-(4-bromophényl)-3-(4-méthanesulfonylphényl)-2-(2-hydroxyéthyl)-2-propénoate d'éthyle**

Formule (XI) : A = phényl, $Y_1$ = 4-Br, $Y_2$ = H

Préparé selon le mode opératoire de l'exemple 12.
Huile utilisée telle quelle pour la suite.

**Exemple 100 : (Z)-3-[1-(4-bromophényl)-1-(4-méthanesulfonylphényl) méthylidène]-dihydro-furan-2-one**

Isomère (Z) : Formule (I) :A = B = phényl, $Y_1$ = 4-Br, $Y_2$ = H,

$X_1$ = 4-$SO_2CH_3$, $X_2$=H,$R_1R_2$ = O, $R_3$ = $R_4$ = H

Préparé selon le mode opératoire de l'exemple 65, purifié par chromatographie dans un mélange dichlorométhane/acétone (10/0.3).
Cristaux de point de fusion 204°C.

**Exemple 101 : 2-(4-méthylthiobenzoyl)furane**

Formule (III) : A = 2-furyl, $Y_1$ = $Y_2$ = H

Préparé selon le mode opératoire de l'exemple 1.
Cristaux de point de fusion 86°C.

**Exemple 102 : 2-(4-méthanesulfonylbenzoyl)furane**

Formule (IV) : A = 2-furyl, $Y_1$ = $Y_2$ = H

Préparé selon le mode opératoire de l'exemple 39.
Cristaux de point de fusion 112°C.

**Exemple 103 : Acide 3-éthoxycarbonyl-4-(furan-2-yl)-4-(4-méthane sulfonylphényl)-3-buténoïque**

Formule (X) : A = 2-furyl, $Y_1$ = $Y_2$ = H

Préparé selon le mode opératoire de l'exemple 11.
Huile utilisée telle quelle pour la suite.

**Exemple 104 : 3-(furan-2-yl)-3-(4-méthanesulfonylphényl)-2-(2-hydroxyéthyl)-2-propénoate d'éthyle**

Formule (XI) : A = 2-furyl, $Y_1 = Y_2 = H$

Préparé selon le mode opératoire de l'exemple 12.
Huile utilisée telle quelle pour la suite.

**Exemple 105 :(Z)-3-[1-(furan-2-yl)-1-(4-méthanesulfonylphényl) méthylidène]-dihydro-furan-2-one**

Isomère (Z) : Formule (I) :A = 2-furyl, B = phényl, $Y_1 = Y_2 = H$,

$X_1 = 4\text{-}SO_2CH_3$, $X_2 = H$, $R_1R_2 = O$, $R_3 = R_4 = H$

Préparé selon le mode opératoire de l'exemple 65, purifié par chromatographie dans un mélange dichlorométhane/acétone (10/0.3).
Cristaux de point de fusion 170°C.

**Exemple 106 : (4-méthylthiobenzoyl)cyclohexane**

Formule (III) : A = cyclohexyl, $Y_1 = Y_2 = H$

Préparé selon le mode opératoire de l'exemple 1.
Cristaux de point de fusion 110°C.

**Exemple 107 : (4-méthanesulfonylbenzoyl)cyclohexane**

Formule (IV) : A = cyclohexyl, $Y_1 = Y_2 = H$

Préparé selon le mode opératoire de l'exemple 39.
Cristaux de point de fusion 116°C.

**Exemple 108 : Acide 3-éthoxycarbonyl-4-cyclohexyl-4-(4-méthane sulfonylphényl)-3-buténoïque**

Formule (X) : A = cyclohexyl, $Y_1 = Y_2 = H$

Préparé selon le mode opératoire de l'exemple 11.
Huile utilisée telle quelle pour la suite.

**Exemple 109 : 3-cyclohexyl-3-(4-méthanesulfonylphényl)-2-(2-hydroxyéthyl)-2-propénoate d'éthyle**

Formule (XI) : A = cyclohexyl, $Y_1 = Y_2 = H$

Préparé selon le mode opératoire de l'exemple 12.
Huile utilisée telle quelle pour la suite.

**Exemple 110 : (Z)-3-[1-cyclohexyl-1-(4-méthanesulfonylphényl)méthylidènel-dihydro-furan-2-one**

Isomère (Z) : Formule (I) : A = cyclohexyl, B = phényl, $Y_1 = Y_2 = H$,

$X_1 = 4\text{-}SO_2CH_3$, $X_2 = H$, $R_1R_2 = O$, $R_3 = R_4 = H$

Préparé selon le mode opératoire de l'exemple 65.
Cristaux de point de fusion 148°C.

**Exemple 111 : 3-fluoro-4-méthyl-4'-méthylthiobenzophénone**

Formule (III) : A = phényl, $Y_1 = 3\text{-}F$, $Y_2 = 4\text{-}CH_3$

A une suspension de 6.4 g de magnésium en copeaux dans 5 ml d'éther éthylique anhydre est ajoutée goutte à goutte une solution de 50 g de 4-bromo-2-fluorotoluène dans 150 ml d'éther éthylique anhydre. A la fin de l'addition, le mélange est agité 30 minutes puis une solution de 4-méthylthiobenzonitrile dans 200 ml de tétrahydrofurane anhydre est ajoutée. L'éther éthylique est distillé et le mélange est ensuite chauffé au reflux pendant 4 heures. Après retour à température ambiante, 300 ml d'acide chlorhydrique 6N sont ajoutés goutte à goutte. Le mélange est ensuite chauffé au reflux pendant 6 heures puis refroidi à température ambiante et extrait à l'éther isopropylique. La phase organique est séchée sur sulfate de magnésium puis évaporée à sec. Le résidu cristallise dans l'éther éthylique pour donner 25 g de 3-fluoro-4-méthyl-4'-méthylthio benzophénone sous forme de cristaux de point de fusion 94°C.

**Exemple 112 : 3-fluoro-4-méthyl-4'-méthanesulfonylbenzophénone**

Formule (IV) : A = phényl, $Y_1 = 3\text{-}F$, $Y_2 = 4\text{-}CH_3$

Préparé selon le mode opératoire de l'exemple 39.
Cristaux de point de fusion 170°C.

**Exemple 113 : Acide (Z)-3-éthoxycarbonyl-4-(3-fluoro-4-méthylphényl)-4-(4-méthanesulfonylphényl)-3-buténoïque**

Formule (X) : A = phényl, $Y_1 = 3\text{-}F$, $Y_2 = 4\text{-}CH_3$

Préparé selon le mode opératoire de l'exemple 11, purifié par traitement du mélange brut des deux isomères (E) et (Z) avec 1 équivalent de D-(+)-α-méthylbenzylamine dans 5 volumes d'acétate d'éthyle. Les cristaux formés

sont éliminés (sel de l'isomère (E)) et le filtrat est acidifié par l'acide chlorhydrique dilué, décanté et évaporé sous vide pour donner une huile correspondant à l'isomère (Z) contenant moins de 5 % d'isomère (E).

**Exemple 114 : (Z)-3-(3-fluoro-4-méthylphényl)-3-(4-méthanesulfonylphényl)-2-(2-hydroxyéthyl)-2-propénoate d'éthyle**

Formule (XI) : A = phényl, $Y_1$ = 3-F, $Y_2$ = 4-$CH_3$

Préparé selon le mode opératoire de l'exemple 12.
Huile utilisée telle quelle pour la suite.

**Exemple 115 : (Z)-3-[1-(3-fluoro-4-méthylphényl)-1-(4-méthanesulfonyl phényl)méthylidène]-dihydro-furan-2-one**

Isomère (Z) : Formule (I) : A = B = phényl, $Y_1$ = 3-F, $Y_2$ = 4-$CH_3$,

$X_1$ = 4-$SO_2CH_3$, $X_2$ = H, $R_1R_2$ = O, $R_3$ = $R_4$ = H

Préparé selon le mode opératoire de l'exemple 65.
Cristaux de point de fusion 169-170°C.

**Exemple 116 : 4-trifluorométhyl-4'-méthylthiobenzophénone**

Formule (III) :A = phényl, $Y_1$ = 4-$CF_3$, $Y_2$ = H,

Préparé selon le mode opératoire de l'exemple 1.
Cristaux de point de fusion 139°C.

**Exemple 117 : 4-trifluorométhyl-4'-méthanesulfonylbenzophénone**

Formule (IV) : A = phényl, $Y_1$ = 4-$CF_3$, $Y_2$ = H

Préparé selon le mode opératoire de l'exemple 39.
Cristaux de point de fusion 138°C.

**Exemple 118 : Acide 3-éthoxycarbonyl-4-(4-trifluorométhylphényl)-4-(4-méthanesulfonylphényl)-3-buténoïque**

Formule (X) : A = phényl, $Y_1$ = 4-$CF_3$, $Y_2$ = H

Préparé selon le mode opératoire de l'exemple 11.
Huile utilisée telle quelle pour la suite.

**Exemple 119 : 3-(4-trifluorométhylphényl)-3-(4-méthanesulfonylphényl)-2-(2-hydroxyéthyl)-2-propénoate d'éthyle**

Formule (XI) : A = phényl, $Y_1$ = 4-$CF_3$, $Y_2$ = H

Préparé selon le mode opératoire de l'exemple 12.
Huile utilisée telle quelle pour la suite.

**Exemple 120 : (E)-3-[1-(4-trifluorométhylphényl)-1-(4-méthanesulfonyl phényl)méthylidène]-dihydro-furan-2-one**

Isomère (E) : Formule (I) : A = B = phényl, $Y_1$ = 4-$CF_3$, $Y_2$ = H,

$X_1$ = 4-$SO_2CH_3$, $X_2$ = H, $R_1R_2$ = O, $R_3$ = $R_4$ = H

Préparé selon le mode opératoire de l'exemple 65, purifié par chromatographie dans un mélange dichlorométhane/acétone (10/0.3).
Cristaux de point de fusion 188-189°C.

**Exemple 121 : 4-benzylthio-4'-chlorobenzophénone**

Préparé selon le mode opératoire de l'exemple 56 à partir du 4-bromochlorobenzène et du 4-benzylthiobenzonitrile préparé à l'exemple 55.
Cristaux de point de fusion 134°C.

**Exemple 122 : 4-t-butylaminosulfonyl-4'-chlorobenzophénone**

Préparé selon le mode opératoire de l'exemple 57.
Cristaux de point de fusion 163°C.

**Exemple 123 : Acide 3-éthoxycarbonyl-4-(4-t-butylaminosulfonylphényl) 4-(4-chlorophényl)-3-buténoïque**

Formule (XII) : A = phényl, $Y_1$ = 4-Cl, $Y_2$ = H

Préparé selon le mode opératoire de l'exemple 58.
Huile utilisée telle quelle pour la suite.

**Exemple 124 : (Z)-3-(4-t-butylaminosulfonylphényl)-3-(4-chlorophényl)-2-(2-hydroxyéthyl)-2-propénoate d'éthyle**

Préparé selon le mode opératoire de l'exemple 12. L'huile obtenue est reprise dans l'éther éthylique et les cristaux formés sont essorés (isomère E). Le filtrat est concentré sous vide et le résidu repris à l'éther de pétrole cristallise pour donner l'isomère (Z) sous forme de cristaux de point de fusion 120°C.

**Exemple 125 : (Z)-4-[(4-chlorophényl)-(2-oxo-dihydro-furan-3-ylidène)-méthyl] benzènesulfonamide**

Formule (I) : A = B = phényl, $Y_1$ = 4-Cl, $Y_2$ = H, $X_1$ = 4-$SO_2NH_2$,

$X_2$ = H, $R_1R_2$ = O, $R_3$ = $R_4$ = H

Une solution de 10 g de (Z)-3-(4-t-butylaminosulfonylphényl)-3-(4-chlorophényl)-2- (2-hydroxyéthyl)-2-propénoate d'éthyle préparé à l'exemple 124, dans 80 ml d'acide trifluoroacétique est chauffée au reflux pendant 15 heures. Après évaporation sous vide du solvant, le résidu est repris au dichlorométhane. La phase organique est lavée à l'eau puis séchée sur sulfate de magnésium et évaporée sous vide pour donner une huile qui cristallise dans un mélange acétone/éther isopropylique pour donner 3.9 g de (Z)-4-[(4-chlorophényl)-(2-oxo-dihydro-furan-3-ylidène)-méthyl] benzènesulfonamide sous forme de cristaux de point de fusion 187-189°C.

**Exemple 126 : 4-benzylthio-3'-fluoro-4'-méthylbenzophénone**

Préparé selon le mode opératoire de l'exemple 56 à partir du 4-bromo-2-fluorotoluène.
Cristaux de point de fusion 122°C.

**Exemple 127 : 4-t-butylaminosulfonyl-3'-fluoro-4'-méthylbenzophénone**

Préparé selon le mode opératoire de l'exemple 57.
Cristaux de point de fusion 132°C.

**Exemple 128 : Acide (Z)-3-éthoxycarbonyl-4-(4-t-butylaminosulfonylphényl)-4-(3-fluoro-4-méthylphényl)-3-buténoïque**

Formule (XII) : A = phényl, $Y_1$ = 3-F, $Y_2$ = 4-$CH_3$

Préparé selon le mode opératoire de l'exemple 58, l'huile obtenue étant reprise dans le t-butyl-méthyl éther et les cristaux formés étant essorés pour donner l'isomère (E) de point de fusion 96°C. Le filtrat est concentré sous vide pour donner l'isomère (Z) sous forme d'une huile utilisée telle quelle pour la suite.

**Exemple 129 : (Z)-3-(4-t-butylaminosulfonylphényl)-3-(3-fluoro-4-méthyl phényl)-2-(2-hydroxyéthyl)-2-propénoate d'éthyle**

Préparé selon le mode opératoire de l'exemple 12 à partir de l'isomère (Z) de l'acide de l'exemple 128.
Huile utilisée telle quelle pour la suite.

**Exemple 130 : (Z)-4-[(3-fluoro-4-méthylphényl)-(2-oxo-dihydro-furan-3-ylidène)méthyl]benzènesulfonamide**

Formule (I) : A = B = phényl, $Y_1$ = 3-F, $Y_2$ = 4-$CH_3$,

$X_1$ = 4-$SO_2NH_2$, $X_2$ = H, $R_1R_2$ = O, $R_3$ = $R_4$ = H

Préparé selon le mode opératoire de l'exemple 125.
Cristaux de point de fusion 170-172°C.

**Exemple 131 : 4-benzylthio-4'-fluoro-3'-méthylbenzophénone**

Préparé selon le mode opératoire de l'exemple 56 à partir du 5-bromo-2-fluorotoluène.

Cristaux de point de fusion 123°C.

**Exemple 132 : 4-t-butylaminosulfonyl-4'-fluoro-3'-méthylbenzophénone**

Préparé le mode opératoire de l'exemple 57.

Cristaux de point de fusion 108°C.

**Exemple 133 : Acide (Z)-3-éthoxycarbonyl-4-(4-t-butylaminosulfonylphenyl)-4-(4-fluoro-3-méthylphényl)-3-buténoïque**

Formule (XII) : A = phényl, $Y_1$ = 3-$CH_3$, $Y_2$ = 4-F

Préparé selon le mode opératoire de l'exemple 58, l'huile obtenue étant reprise dans le t-butyl-méthyl éther et les cristaux formés étant éliminés (isomère (E)). Le filtrat est évaporé sous vide pour donner l'isomère (Z) sous forme d'huile utilisée telle quelle pour la suite.

**Exemple 134 : (Z)-3-(4-t-butylaminosulfonylphényl)-3-(4-fluoro-3-méthyl phényl)-2-(2-hydroxyéthyl)-2-propénoate d'éthyle**

Préparé selon le mode opératoire de l'exemple 12.

Cristaux de point de fusion 128°C.

**Exemple 135 : (Z)-4-[(4-fluoro-3-méthylphényl)-(2-oxo-dihydro-furan-3-ylidène)méthyl]benzènesulfonamide**

Formule (I) : A = B = phényl, $Y_1$ = 3-$CH_3$, $Y_2$ = 4-F,

$X_1$ = 4-$SO_2NH_2$, $X_2$ = H, $R_1R_2$ = O, $R_3$ = $R_4$ = H

Préparé selon le mode opératoire de l'exemple 125.

Cristaux de point de fusion 228-229°C.

**Exemple 136 : (E)-3-(4-t-butylaminosulfonylphényl)-3-(3-fluoro-4-méthyl phényl)2-(2-hydroxyéthyl)-3-propénoate d'éthyle**

Préparé selon le mode opératoire de l'exemple 12, à partir de l'isomère (E) de l'acide de l'exemple 128.

Huile utilisée telle quelle pour la suite.

**Exemple 137 : Acide (E)-3-(4-t-butylaminosulfonylphényl)-3-(3-fluoro-4-méthylphényl)-2-(2-hydroxyéthyl)-3-propénoïque**

Une solution de 16 g de (E)-3-(4-t-butylaminosulfonylphényl)-3-(3-fluoro-4-méthylphényl)-2-(2-hydroxyéthyl)-3-propénoate d'éthyle, préparé à l'exemple 136, dans 50 ml d'éthanol, contenant 3 g de soude et 5 ml d'eau, est chauffée au reflux pendant 2 heures. Le mélange est concentré sous vide, repris à l'eau, lavé à l'éther éthylique puis acidifié par l'acide chlorhydrique dilué et extrait au dichlorométhane. La phase organique est séchée sur sulfate de magnésium, puis évaporée à sec sous vide pour donner un résidu qui cristallise dans l'éther isopropylique pour donner 7.6 g d'acide (E)- 3-(4-t-butylamino sulfonylphényl)-3-(3-fluoro-4-méthylphényl)-2-(2-hydroxyéthyl)-3-propénoïque sous forme de cristaux de point de fusion 160°C.

**Exemple 138 : (E)-2-[1-(4-t-butylaminosulfonylphényl)-1-(3-fluoro-4-méthyl phényl)méthylidène]butane-1,4-diol**

A une solution de 7.8 g d'acide (E)- 3-(4-t-butylaminosulfonylphényl)-3-(3-fluoro-4-méthylphényl)-2-(2-hydroxyéthyl)-3-propénoïque préparé à l'exemple 137, dans 50 ml de tétrahydrofurane anhydre, sont ajoutés goutte à goutte 5 ml de complexe borane/sulfure de méthyle. Le mélange est agité 6 heures à température ambiante puis 10 ml de méthanol sont ajoutés goutte à goutte. Le mélange est concentré sous vide, repris avec une solution aqueuse de carbonate de potassium et extrait à l'éther éthylique. La phase organique est séchée sur sulfate de magnésium et évaporée sous vide pour donner 6.3 g de (E) -2-[1-(4-t-butylaminosulfonylphényl)-1-(3-fluoro-4-méthylphényl)méthylidène]butane-1,4-diol sous forme de cristaux de point de fusion 106°C.

**Exemple 139 : (E)-4-[(3-fluoro-4-méthylphényl)-(tétrahydro-furan-3-ylidène) méthyl]benzènesulfonamide**

Formule (I) : A = B = phényl, $X_1$ = 3-F, $X_2$ = 4-$CH_3$,

$Y_1$ = 4-$SO_2NH_2$, $Y_2$ = H, $R_1$ = $R_2$ = $R_3$ = $R_4$ = H

Une solution de 6.3 g de (E) -2-[1-(4-t-butylaminosulfonylphényl)-1-(3-fluoro-4-méthylphényl)méthylidène]butane-1,4-diol préparé à l'exemple 138 dans 75 ml d'acide trifluoroacétique est chauffée 10 heures au reflux. Le solvant est concentré sous vide et le résidu est repris dans une solution de soude diluée, lavée au dichlorométhane puis acidifiée par l'acide chlorhydrique dilué et extrait au dichlorométhane. Les phases organiques sont réunies et séchées sur sulfate de magnésium puis évaporées sous vide. Le résidu est organisé dans le t-butyl-méthyl éther pour donner 4 g de (E)-4-[(3-fluoro-4-méthylphényl)-(tétrahydro-furan-3-ylidène)méthyl]benzènesulfonamide sous forme de cristaux de point de fusion 174-176°C.

**Exemple 140 : 4-benzylthio-3'-chloro-4'-fluorobenzophénone**

Préparé selon le mode opératoire de l'exemple 56, à partir du 4-bromo-2-chlorofluorobenzène.
Cristaux de point de fusion 102°C.

**Exemple 141 : 4-t-butylaminosulfonyl-3'-chloro-4'-fluorobenzophénone**

Préparé selon le mode opératoire de l'exemple 57.
Cristaux de point de fusion 108°C.

**Exemple 142 : Acide 3-éthoxycarbonyl-4-(4-t-butylaminosulfonylphényl)-4-(3-chloro-4-fluorophényl)-3-buténoïque**

Formule (XII) : A = phényl, $Y_1$ = 3-Cl, $Y_2$ = 4-F

Préparé selon le mode opératoire de l'exemple 58.
Huile utilisée telle quelle pour la suite.

**Exemple 143 : (Z)-4-[(3-chloro-4-fluorophényl)-(2-oxo-dihydro-furan-3-ylidène)méthyl]benzènesulfonamide**

Formule (I) : A = B = phényl, $Y_1$ = 3-Cl, $Y_2$ = 4-F, $X_1$ = 4-$SO_2NH_2$,

$X_2$ = H, $R_1R_2$ = O, $R_3$ = $R_4$ = H

A une solution de 17 g d'acide 3-éthoxycarbonyl-4-(4-t-butylamino sulfonylphényl)-4-(3-chloro-4-fluorophényl)-3-buténoïque préparé à l'exemple 142 dans 150 ml de tétrahydrofurane sont ajoutés goutte à goutte 7 ml de complexe borane / sulfure de méthyle. Le mélange est agité 6 heures à température ambiante puis 30 ml d'éthanol sont ajoutés goutte à goutte. Après addition d'une solution aqueuse de carbonate de potassium, le mélange est extrait au dichloro méthane. La phase organique est séchée et évaporée sous vide. Le résidu (13,8 g) est repris dans 30 ml d'éthanol contenant une solution de 3 g de soude dans 10 ml d'eau et le mélange est chauffé à 70°C pendant 3 heures. Les solvants sont concentrés sous vide et le résidu est repris à l'eau ; la phase aqueuse est lavée à l'éther éthylique, acidifiée à l'acide chlorhydrique et extraite au dichlorométhane, séchée sur sulfate de magnésium et évaporée sous vide. Le résidu est repris à l'éther éthylique et les cristaux obtenus sont essorés pour donner 3 g d'acide (E)-3-(4-t-butylaminosulfonylphényl)-3-(3-fluoro-4-chlorophényl)-2-(2-hydroxy éthyl)-3-propénoïque (point de fusion 180°C). Le filtrat est concentré sous vide et le résidu est repris dans 60 ml d'acide trifluoroacétique. Le mélange est chauffé 17 heures au reflux puis concentré sous vide. Le résidu est repris avec une solution diluée de soude et extrait au dichlorométhane. La phase organique est séchée sur sulfate de magnésium puis évaporée sous vide pour donner 2 g de (Z)-4-[(3-chloro-4-fluorophényl)-(2-oxo-dihydro-furan-3-ylidène)méthyl]benzène sulfonamide sous forme de cristaux de point de fusion 244-246°C.

**Exemple 144 : 4-benzylthio-3'-fluoro-4'-méthoxybenzophénone**

Préparé selon le mode opératoire de l'exemple 56 à partir du 4-bromo-2-fluoroanisole.
Cristaux de point de fusion 125°C.

**Exemple 145 : 4-t-butylaminosulfonyl-3'-fluoro-4'-méthoxybenzophénone**

Préparé selon le mode opératoire de l'exemple 57.
Cristaux de point de fusion 136°C.

**Exemple 146 : Acide (Z)-3-éthoxycarbonyl-4-(4-t-butylaminosulfonylphényl)-4-(3-fluoro-4-méthoxyphényl)-3-buténoïque**

Formule (XII) : A = phényl, $Y_1$ = 3-F, $Y_2$ = 4-OMe

Préparé selon le mode opératoire de l'exemple 58, l'huile obtenue est reprise par le t-butyl méthyl éther pour donner l'isomère (Z) sous forme de cristaux amorphes utilisés tels quels pour la suite.

**Exemple 147 : (Z)-3-(4-t-butylaminosulfonylphényl)-3-(3-fluoro-4-méthoxy phényl)-2-(2-hydroxyéthyl)-2-propénoate d'éthyle**

Préparé selon le mode opératoire de l'exemple 12.
Huile utilisée telle quelle pour la suite.

**Exemple 148 : (Z)-4-[(3-fluoro-4-méthoxyphényl)-(2-oxo-dihydro-furan-3-ylidène)méthyl]benzènesulfonamide**

Formule (I) : A = B = phényl, $Y_1$ = 3-F, $Y_2$ = 4-OMe,

$X_1$ = 4-$SO_2NH_2$, $X_2$ = H, $R_1R_2$ = O, $R_3$ = $R_4$ = H

Préparé selon le mode opératoire de l'exemple 125.
Cristaux de point de fusion 149-150°C.

**Exemple 149 : 3-(4-fluorobenzoyl)pyridine**

A une suspension de 140 g de chlorhydrate de chlorure de l'acide nicotinique dans 500 ml de fluorobenzène,

refroidie à 0°C, sont ajoutés par portions 280 g de chlorure d'aluminium. Le mélange est porté au reflux 6 heures puis refroidi et versé sur de la glace. Après addition de soude jusqu'à pH = 8, le mélange est extrait au dichlorométhane. La phase organique est séchée sur sulfate de magnésium puis évaporée sous vide. Le résidu cristallise dans un mélange pentane/éther isopropylique pour donner 108.5 g de 3-(4-fluorobenzoyl)pyridine sous forme de cristaux de point de fusion 91°C.

**Exemple 150 : 3-(4-benzylthiobenzoyl)pyridine**

A une solution de 43 g de benzylmercaptan dans 500 ml de N,N-diméthyl formamide sont ajoutés 14 g d'hydrure de sodium à 60 %. Le mélange est agité 20 minutes à température ambiante et 70 g de 3-(4-fluorobenzoyl) pyridine, préparée à l'exemple 149, sont ajoutés. Le mélange est chauffé 8 heures à 80°C puis concentré sous vide et le résidu est repris à l'eau. Les cristaux formés sont essorés, dissous dans du dichlorométhane, la solution est séchée sur sulfate de magnésium et concentrée sous vide. Le résidu cristallise dans un mélange pentane/éther isopropylique pour donner 81 g de 3-(4-benzylthiobenzoyl)pyridine sous forme de cristaux de point de fusion 102°C.

**Exemple 151 : 3-(4-t-butylaminosulfonylbenzoyl)pyridine**

Préparé selon le mode opératoire de l'exemple 57.
Cristaux de point de fusion 179°C.

**Exemple 152 : Acide (Z)-3-éthoxycarbonyl-4-(4-t-butylaminosulfonylphényl)-4-(3-pyridyl)-3-buténoïque**

Formule (XII) : A = 3-pyridyl, $Y_1 = Y_2 = H$

Préparé selon le mode opératoire de l'exemple 58, l'huile obtenue étant reprise dans l'acétate d'éthyle chaud, les cristaux formés étant essorés (isomère (E) de point de fusion 209°C). Le filtrat est évaporé et le résidu repris au pentane cristallise pour donner l'isomère (Z) sous *forme* de cristaux de point de fusion 195°C.

**Exemple 153 : (Z)-3-(4-t-butylaminosulfonylphényl)-3-(3-pyridyl)-2-(2-hydroxyéthyl)-2-propénoate d'éthyle**

Préparé selon le mode opératoire de l'exemple 12.
Cristaux amorphes utilisés tels quels pour la suite.

**Exemple 154 : (Z)-[(3-pyridyl)-(2-oxo-dihydro-furan-3-ylidène)méthyl] benzènesulfonamide**

Formule (I) : A = 3-pyridyl, B = phényl, $X_1$ = 4-$SO_2NH_2$,

$X_2 = H$, $Y_1 = Y_2 = H$, $R_1R_2 = O$, $R_3 = R_4 = H$

Préparé selon le mode opératoire de l'exemple 125.
Cristaux de point de fusion 219-220°C.

**Exemple 155 : 3-chloro-4-méthoxy-4'-méthylthiobenzophénone**

Formule (III) : A = phényl, $Y_1$ = 3-Cl, $Y_2$ = 4-OMe

Préparé selon le mode opératoire de l'exemple 1.
Cristaux de point de fusion 100°C.

**Exemple 156 : 3-chloro-4-méthoxy-4'-méthanesulfonylbenzophénone**

Formule (IV) : A = phényl, $Y_1$ = 3-Cl, $Y_2$ = 4-OMe

Préparé selon le mode opératoire de l'exemple 39.
Cristaux de point de fusion 164°C.

**Exemple 157 : Acide (Z)-3-éthoxycarbonyl-4-(3-chloro-4-méthoxyphényl)-4-(4-méthanesulfonylphényl)-3-buténoïque**

Formule (X) : A = phényl, $Y_1$ = 3-Cl, $Y_2$ = 4-OMe

Préparé selon le mode opératoire de l'exemple 11, l'huile obtenue étant reprise dans l'éther et les cristaux formés étant essorés pour donner l'isomère (Z) sous forme de cristaux de point de fusion 179°C.

**Exemple 158 : (Z)-3-(3-chloro-4-méthoxyphényl)-3-(4-méthanesulfonyl phényl)-2-(2-hydroxyéthyl)-3-propénoate d'éthyle**

Formule (XI) : A = phényl, $Y_1$ = 3-Cl, $Y_2$ = 4-OMe

Préparé selon le mode opératoire de l'exemple 12.
Cristaux de point de fusion 102°C.

**Exemple 159 : (Z)-3-[1-(3-chloro-4-méthoxyphényl)-1-(4-méthanesulfonyl phényl)méthylidène]-dihydro-furan-2-one**

Formule (I) : A = B = phényl, $Y_1$ = 3-Cl, $Y_2$ = 4-OMe,

$X_1$ = 4-$SO_2CH_3$, $X_2$ = H, $R_1R_2$ = O, $R_3$ = $R_4$ = H

Préparé selon le mode opératoire de l'exemple 65.
Cristaux de point de fusion 177°C.

**Exemple 160 : 2-(4-méthylthiobenzoyl)thiophène**

Formule (III) : A = 2-thiényl, $Y_1$ = $Y_2$ = H

Préparé selon le mode opératoire de l'exemple 1.
Cristaux de point de fusion 60°C.

**Exemple 161 : 2-(4-méthanesulfonylbenzoyl)thiophène**

Formule (IV) : A = 2-thiényl, $Y_1 = Y_2 = H$

Préparé selon le mode opératoire de l'exemple 39.
Cristaux de point de fusion 140°C.

**Exemple 162 : Acide (E)-3-éthoxycarbonyl-4-(4-méthanesulfonylphényl)-4-(2-thiényl)-3-buténoïque**

Formule (X) : A = 2-thiényl, $Y_1 = Y_2 = H$

Préparé selon le mode opératoire de l'exemple 11.
Cristaux de point de fusion 120°C.

**Exemple 163 : (E)-3-(4-méthanesulfonylphényl)-3-(2-thiényl)-2-(2-hydroxy éthyl)-2-propénoate d'éthyle**

Formule (XI) : A = 2-thiényl, $Y_1 = Y_2 = H$

Préparé selon le mode opératoire de l'exemple 12.
Cristaux de point de fusion 116°C.

**Exemple 164 : (E)-3-[1-(4-méthanesulfonylphényl)-1-(2-thiényl)méthylidène]-dihydro-furan-2-one**

Formule (I) : A = 2-thiényl, B = phényl, $Y_1 = Y_2 = H$,

$X_1 = 4\text{-}SO_2CH_3$, $X_2 = H$, $R_1R_2 = O$, $R_3 = R_4 = H$

Préparé selon le mode opératoire de l'exemple 65.
Cristaux de point de fusion 246°C.

**Exemple 165 : 4-(2-naphtoyl)méthylthiobenzène**

Formule (III) : A = 2-naphtyl, $Y_1 = Y_2 = H$

Préparé selon le mode opératoire de l'exemple 1.
Cristaux de point de fusion 98°C.

**Exemple 166 : 4-(2-naphtoyl)méthanesulfonylbenzène**

Formule (IV) : A = 2-naphtyl, $Y_1 = Y_2 = H$

Préparé selon le mode opératoire de l'exemple 39.
Cristaux de point de fusion 150°C.

**Exemple 167 : Acide-3-éthoxycarbonyl-4-(2-naphtyl)-4-(4-méthanesulfonyl phényl)-3-buténoïque**

Formule (X) : A = 2-naphtyl, $Y_1 = Y_2 = H$

Préparé selon le mode opératoire de l'exemple 11.
Huile utilisée telle quelle pour la suite.

**Exemple 168 : 3-(2-naphtyl)-3-(4-méthanesulfonylphényl)-2-(2-hydroxy éthyl)-2-propénoate d'éthyle**

Formule (XI) : A = 2-naphtyl, $Y_1 = Y_2 = H$

Préparé selon le mode opératoire de l'exemple 12.
Huile utilisée telle quelle pour la suite.

**Exemple 169 : (Z)-3-[1-(2-naphtyl)-1-(4-méthanesulfonylphényl) méthylidène]-dihydro-furan-2-one**

Formule (I) : A = 2-naphtyl, B = phényl, $Y_1 = Y_2 = H$,

$X_1 = 4\text{-}SO_2CH_3$, $X_2 = H$, $R_1R_2 = O$, $R_3 = R_4 = H$

Préparé selon le mode opératoire de l'exemple 65, purifié par chromatographie dans un mélange dichlorométhane / acétone (10/0.3).
Cristaux de point de fusion 244°C.

**Exemple 170 : 3,5-dichloro-4'-fluorobenzophénone**

Un mélange de 50 g de chlorure de 3,5-dichlorobenzoyle et de 150 ml de fluorobenzène est refroidi à 0°C et 65 g de trichlorure d'aluminium sont ajoutés par portion. Le mélange est ensuite agité à température ambiante pendant 2 heures puis au reflux pendant 4 heures. Après refroidissement, le mélange est versé sur un mélange glace / acide chlorhydrique dilué et extrait au dichlorométhane. La phase organique est séchée sur sulfate de magnésium et évaporée à sec pour donner 59 g de 3,5-dichloro-4'-fluorobenzophénone sous forme de cristaux de point de fusion 65°C.

**Exemple 171 : 3,5-dichloro-4-'benzylthiobenzophénone**

Préparé selon le mode opératoire de l'exemple 150, à partir de la 3,5-dichloro-4'-fluorobenzophénone préparée à l'exemple 170.
Cristaux de point de fusion 80°C.

**Exemple 172 : 4-t-butylaminosulfonyl-3',5'-dichlorobenzophénone**

Préparé selon le mode opératoire de l'exemple 57 à partir de la 3,5-dichloro-4'-benzylthiobenzophénone préparée à l'exemple 171.
Cristaux de point de fusion 144°C.

**Exemple 173 : Acide 3-éthoxycarbonyl-4-(4-t-butylaminosulfonylphényl)-4-(3,5-dichlorophényl)-3-buténoïque**

Formule (XII) : A = phényl, $Y_1$ = 3-Cl, $Y_2$ = 5-Cl

Préparé selon le mode opératoire de l'exemple 58.
Huile utilisée telle quelle pour la suite.

**Exemple 174 : 3-(4-t-butylaminosulfonylphényl)-3-(3,5-dichlorophényl)-2-(2-hydroxyéthyl)-2-propénoate d'éthyle**

Préparé selon le mode opératoire de l'exemple 12.
Huile utilisée telle quelle pour la suite.

**Exemple 175 : (Z)-4-[(3,5-dichlorophényl)-(2-oxo-dihydro-furan-3-ylidène) méthyl]benzènesulfonamide**

Isomère (Z) : Formule (I) : A = B = phényl, $Y_1$ = 3-Cl, $Y_2$ = 5-Cl,

$X_1$ = 4-$SO_2NH_2$, $X_2$ = H, $R_1R_2$ = O, $R_3$ = $R_4$ = H

Préparé selon le mode opératoire de l'exemple 65. Les cristaux obtenus sont chromatographiés sur gel de silice dans un mélange dichlorométhane/acétone (9/1) pour donner après recristallisation dans l'acide acétique , le (Z)-4-[(3,5-dichlorophényl)-(2-oxo-dihydro-furan-3-ylidène)méthyl]benzènesulfonamide sous forme de cristaux de point de fusion 217°C.

**Exemple 176 : 3-chloro-4'-fluorobenzophénone**

Préparé selon le mode opératoire de l'exemple 170.
Cristaux de point de fusion 76°C.

**Exemple 177 : 3-chloro-4'-benzylthiobenzophénone**

Préparé selon le mode opératoire de l'exemple 150 à partir de la 3-chloro-4'-fluorobenzophénone préparée à l'exemple 176.
Cristaux de point de fusion 90°C.

**Exemple 178 : 4-t-butylaminosulfonyl-3'-chlorobenzophénone**

Préparé selon le mode opératoire de l'exemple 57 à partir de la 3'-chloro-4'-benzylthiobenzophénone, préparée à l'exemple 177.
Cristaux de point de fusion 128°C.

**Exemple 179 : Acide 3-éthoxycarbonyl-4-(4-t-butylaminosulfonylphényl)-4-(3-chlorophényl)-3-buténoïque**

Formule (XII) : A = phényl, $Y_1$ = 3-Cl, $Y_2$ = H

Préparé selon le mode opératoire de l'exemple 58.
Huile orangée utilisée telle quelle pour la suite.

**Exemple 180 : 3-(4-t-butylaminosulfonylphényl)-3-(3-chlorophényl)-2-(2-hydroxyéthyl)-2-propénoate d'éthyle**

Préparé selon le mode opératoire de l'exemple 12.
Huile utilisée telle quelle pour la suite.

**Exemple 181 : (Z)-4-[(3-chlorophényl)-(2-oxo-dihydro-furan-3-ylidène) méthyl]benzènesulfonamide**

Isomère (Z) : Formule (I) : A = B = phényl, $Y_1$ = 3-Cl, $Y_2$ = H,

$X_1$ = 4-$SO_2NH_2$, $X_2$ = H, $R_1R_2$ = O, $R_3$ = $R_4$ = H

Préparé selon le mode opératoire de l'exemple 65, purifié par chromatographie sur gel de silice avec un mélange dichlorométhane / acétone (10/1).
Cristaux de point de fusion 178°C.

**Exemple 182 : 4-fluoro-3'-méthylbenzophénone**

Préparé selon le mode opératoire de l'exemple 170.
Cristaux de point de fusion 50°C.

**Exemple 183 : 4-benzylthio-3'-méthylbenzopéhnone**

Préparé selon le mode opératoire de l'exemple 150, à partir de la 4-fluoro-3-'méthylbenzophénone, préparée à l'exemple 182.
Cristaux de point de fusion 98°C.

**Exemple 184: 4-t-butylaminosulfonyl-3'-méthylbenzophénone**

Préparé selon le mode opératoire de l'exemple 57, à partir de la 4-benzylthio-3'-méthylbenzophénone, préparée à l'exemple 183.
Cristaux de point de fusion 116°C.

**Exemple 185 : Acide 3-éthoxycarbonyl-4-(4-t-butylaminosulfonylphényl)-4-(3-méthylphényl)-3-buténoïque**

Formule (XII) : A = phényl, $Y_1$ = 3-$CH_3$, $Y_2$ = H

Préparé selon le mode opératoire de l'exemple 58.
Huile utilisée telle quelle pour la suite.

**Exemple 186 : 3-(4-t-butylaminosulfonylphényl)-3-(3-méthylphényl)-2-(2-hydroxyéthyl)-2-propénoate d'éthyle**

Préparé selon le mode opératoire de l'exemple 12.
Huile utilisée telle quelle pour la suite.

**Exemple 187 : (Z)-4-[(3-méthylphényl)-(2-oxo-dihydro-furan-3-ylidène) méthyl]benzènesulfonamide**

Isomère (Z) : Formule (I) : A = B = phényl, $Y_1$ = 3-$CH_3$, $Y_2$ = H,

X1 = 4-SO2NH2, X2 = H, R1R2 = O, R3 = R4 = H

Préparé selon le mode opératoire de l'exemple 65. L'huile obtenue est chromatographiée sur gel de silice dans un mélange dichlorométhane / acétone 10/1 pour donner le (Z)-4-[(3-méthylphényl)-(2-oxo-dihydro-furan-3-ylidène) méthyl]benzènesulfonamide sous forme de cristaux de point de fusion 166°C.

**Exemple 188 : 3-chloro-4-méthoxy-4'-benzylthiobenzophénone**

Préparé selon le mode opératoire de l'exemple 56 à partir du 2-chloro-4-bromoanisole.
Cristaux de point de fusion 115°C.

**Exemple 189 : 4-t-butylaminosulfonyl-3'-chloro-4'-méthoxybenzophénone**

Préparé selon le mode opératoire de l'exemple 57 à partir de la 3-chloro-4-méthoxy-4'-benzylthiobenzophénone.
Cristaux de point de fusion 120°C.

**Exemple 190 : Acide 3-éthoxycarbonyl-4-(4-t-butylaminosulfonylphényl)-4-(3-chloro-4-méthoxyphényl)-3-buténoïque**

Formule (XII) : A = phényl, $Y_1$ = 3-Cl, $Y_2$ = OMe

Préparé selon le mode opératoire de l'exemple 58.
Huile utilisée telle quelle pour la suite.

**Exemple 191 : 3-(4-t-butylaminosulfonylphényl)-3-(3-chloro-4-méthoxy phényl)-2-(2-hydroxyéthyl)-2-propénoate d'éthyle**

Préparé selon le mode opératoire de l'exemple 12.
Huile utilisée telle quelle pour la suite.

**Exemple 192 : (Z)-4-[(3-chloro-4-méthoxyphényl)-(2-oxo-dihydro-furan-3-ylidène)méthyl]benzènesulfonamide et (E) -4-[(3-chloro-4-méthoxyphényl)-(2-oxo-dihydro-furan-3-ylidène) méthyl] benzènesulfonamide**

Formule (I) : A = B = phényl, $Y_1$ = 3-Cl, $Y_2$ = 4-OMe,

$X_1$ = 4-$SO_2NH_2$, $X_2$ = H, $R_1R_2$ = O, $R_3$ = $R_4$ = H

Préparé selon le mode opératoire de l'exemple 65. L'huile obtenue est chromatographiée sur gel de silice dans un mélange dichlorométhane / acétone (10/1) pour donner un mélange de (E) et de (Z)-4-[(3-chloro-4-méthoxyphényl)-(2-oxo-dihydro-furan-3-ylidène)méthyl]benzènesulfonamide sous forme de cristaux amorphes.

**PHARMACOLOGIE**

L'activité anti-inflammatoire des composés des exemples a été évaluée selon la méthode de l'oedème à la carragénine et l'activité antalgique selon la méthode de l'arthrite au kaolin.

**Méthodes**

**Activité anti-inflammatoire :**

L'activité anti-inflammatoire est évaluée chez le rat par le test de l'oedème à la carragénine. Le produit est administré par voie orale à raison de 2,5 ml/100 g (n = 6 animaux par dose) 2 h 30 après une surcharge hydrique par voie orale (2,5 ml /100 g), une heure après l'administration du produit, l'oedème est induit par injection sous-cutanée

plantaire d'une solution aqueuse de carragénine à 2 %. Les résultats sont exprimés sous forme de $DI_{50}$, dose en mg/kg induisant 50 % de diminution du volume de l'oedème, calculée par régression linéaire en utilisant le volume d'oedème maximum obtenu pour chaque produit testé.

**Activité analgésique :**

L'activité analgésique est évaluée chez le rat par le test de l'arthrite au kaolin. Trente minutes après administration intra articulaire d'une suspension aqueuse de kaolin à 10 %, le produit est administré par voie orale à raison de 1 ml/100 g (n = 10 animaux par dose). Les résultats sont exprimés sous forme de $DE_{50}$, dose en mg/kg induisant 50 % de diminution des cotations maximum obtenues dans le lot contrôle, calculée par régression linéaire.

| Exemple | Activité anti- inflammatoire $DI_{50}$ (mg/kg) | Activité analgésique $DE_{50}$ (mg/kg) |
|---|---|---|
| 4(isomère E) | 1.8 | 7.6 |
| 8(isomère E) | > 30 | - |
| 8 et 65(isomèreZ) | 3.7 | 2.1 |
| 13(isomère Z) | 3.9 | 14.1 |
| 18(isomère Z) | >30 | 6.1 |
| 23(isomère Z) | 9.3 | 7.2 |
| 28(isomère E) | 10.1 | 26.1 |
| 31(isomère E) | 3.1 | 14.2 |
| 34 (isomère E) | - | 38.8 |
| 49(isomère Z) | > 30 | > 30 |
| 54(isomère Z) | >30 | 10.8 |
| 60(isomère E) | 1.7 | 20.9 |
| 70(isomère Z) | 5.5 | 21.5 |
| 75(isomère Z) | 4.6 | - |
| 80(isomère Z) | 4.8 | 5.8 |
| 85(isomère E) | > 30 | > 30 |
| 90(isomère Z) | 17.2 | - |
| 95(isomère Z) | 1.6 | 5.8 |
| 100(isomère Z) | 3.6 | - |
| 125(isomère Z) | 1.8 | 8.5 |
| 130(isomère Z) | 2.0 | 2.4 |
| 135(isomère Z) | 3.3 | 6.1 |
| 143(isomère Z) | 0.7 | 6.4 |

**Inhibition des activités enzymatiques COX-1 et COX-2**

La molécule étudiée est préincubée pendant 10 minutes à 25°C avec 2U de COX-1 (enzyme purifiée de vésicules séminales de bélier) ou 1U de COX-2 (enzyme purifiée de placenta de mouton). L'acide arachidonique (6 µM pour la COX-1, 4 µM pour la COX-2) est ajouté dans le milieu réactionnel et une incubation de 5 minutes à 25°C est réalisée. Au terme de l'incubation, la réaction enzymatique est arrêtée par un ajout de HCl 1N et la PGE2 produite est dosée par EIA.

Les résultats sont exprimés sous forme de $CI_{50}$, concentration en nM correspondant à 50 % de l'inhibition de l'activité enzymatique maximale sur la COX-1 et sur la COX-2 (n = 1 à 4 déterminations).

| Exemple | Inhibition de la COX-2 $CI_{50}$ (nM) | Inhibition de la COX-1 $CI_{50}$ (nM) | Selectivity COX-1/COX-2 ratio |
|---|---|---|---|
| 4(E) | 674 ± 46 (n=3) | > 300000 | >445 |
| 8 ou 65 (Z) | 182 ± 25 (n=4) | 171875 ± 22193 (n = 4) | 944 |
| 13 (Z) | 610 | > 100000 | > 164 |
| 18(Z) | 132 ± 32 (n = 2) | 73200 | 555 |
| 23(E) | 627 | > 100000 | > 159 |
| 28(E) | 18354 | > 10000 | >0.5 |
| 31(E) | 780 | > 100000 | > 128 |
| 34(E) | 694 | > 100000 | > 144 |
| 49(Z) | 418 | >100000 | >239 |
| 54(Z) | 710 | > 100000 | > 141 |
| 60(E) | 296 ± 107 (n=2) | 81000 | 274 |
| 70(Z) | 225 | > 100000 | >444 |
| 80(Z) | 518 | > 100000 | > 193 |
| 85(E) | 350 | > 100000 | >286 |
| 90(Z) | 458 | > 100000 | >218 |
| 95(Z) | 205 ± 35 (n=2) | 309500 ± 27500 (n=2) | 1510 |
| 100(Z) | 166 | 62000 | 373 |
| 125(Z) | 60 | 2525 ± 1312 (n=2) | 42 |
| 130(Z) | 131 ± 19 (n = 2) | 45730 | 349 |
| 135(Z) | 245 ± 76 (n=2) | 19700 | 80 |
| 143(Z) | 73 | 12700 | 174 |

**TOLERANCE**

**Tolérance gastrique :**

L'étude de la tolérance gastrique est réalisée chez le rat Charles River de souche CD pesant de 110 à 150 g. Les animaux sont soumis à une diète hydrique 24 h avant l'administration du produit ou du véhicule seul par voie orale à raison de 1 ml/100 g (n = 6 animaux par dose). Six heures après l'administration, les animaux sont sacrifiés, les estomacs sont prélevés et ouverts selon la grande courbure. Le nombre de points et sillons hémorragiques par estomac identifiés macroscopiquement permet d'établir un index d'ulcération (0 : pas de lésion, 1 : 1 à 2 lésions, 2 : 3 à 4 lésions, 3 : 5 à 8 lésions, 4 : 9 à 16 lésions, 5 : plus de 17 lésions) et d'estimer la dose ulcérigène 50 % ($DU_{50}$ = dose exprimée en mg/kg induisant 4 à 5 lésions).

| Exemple | **$DU_{50}$** (limite de confiance) mg/kg |
|---|---|
| 4 (Isomère E) | >1000 |

(suite)

| Exemple | $DU_{50}$ (limite de confiance) mg/kg |
|---|---|
| 8 ou 65(Isomère Z) | > 1000 |
| 125 (Isomère Z) | >1000 |
| indométacine | 8.3 (5.8-11.8) |

**TOXICOLOGIE**

Les premières études de toxicologie réalisées montrent que les produits des exemples n'induisent aucun effet délétère après absorption orale chez le rat de doses pouvant aller jusqu'à 300 mg/kg.

**Revendications**

**1.** Dérivés diarylméthylidène furaniques caractérisés en ce qu'ils répondent à la formule générale (I):

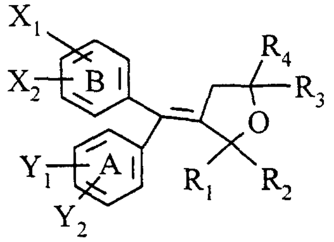

Formule (I)

dans laquelle :

les cycles A et B représentent indépendamment :

- un radical phényle
- un radical naphtyle
- un radical dérivé d'un hétérocycle comportant 5 à 6 chaînons et possédant de 1 à 4 hétéroatomes
- un radical dérivé d'un cycle hydrocarboné saturé ayant de 3 à 7 atomes de carbone

l'un au moins des cycles A et B représentant obligatoirement un radical phényle
au moins l'un des substituants $X_1$, $X_2$, $Y_1$ ou $Y_2$ représente obligatoirement:

- un groupement $-S(O)_n-R$ dans lequel n est un nombre entier égal à 0,1 ou 2 et R est un radical alkyle inférieur de 1 à 6 atomes de carbone, un radical halogéno alkyle inférieur de 1 à 6 atomes de carbone,
- un groupement $-SO_2NH_2$;
  et se trouve en position para sur le radical phényle représenté par A ou B,

les autres représentent indépendamment

- l'atome d'hydrogène
- un atome d'halogène
- un radical alkyle inférieur de 1 à 6 atomes de carbone
- un radical trifluorométhyle,
- un radical O-alkyle inférieur de 1 à 6 atomes de carbone

ou encore

$X_1$ et $X_2$ ou $Y_1$ et $Y_2$ représentent un groupement méthylène dioxy,
$R_1$, $R_2$, $R_3$ et $R_4$ représentent indépendamment

- l'atome d'hydrogène
- un atome d'halogène
- un radical alkyle inférieur de 1 à 6 atomes de carbone,
- un radical halogénoalkyle inférieur de 1 à 6 atomes de carbone,
- un radical aromatique choisi dans le groupe comprenant phényle, naphtyle, thiényle, furyle ou pyridyle,

ou encore

$R_1R_2$ ou $R_3R_4$ représentent un atome d'oxygène
ou bien $R_1$, $R_2$ ou $R_3$, $R_4$, forment ensemble avec l'atome de carbone auquel ils sont attachés un cycle hydrocarboné saturé ayant de 3 à 7 atomes de carbone.

**2.** Dérivés de formule (I) selon la revendication 1, caractérisés en ce que :

les cycles A et B représentent indépendamment un radical :

- phényle
- naphtyle
- pyridyle
- furyle
- thiényle
- cyclohexyle

au moins l'un des substituants $X_1$, $X_2$, $Y_1$ ou $Y_2$ représente obligatoirement un groupement $-SCH_3$, $-SO_2CH_3$ ou $-SO_2NH_2$,
les autres représentent indépendamment :

- l'atome d'hydrogène
- un atome d'halogène
- un radical alkyle inférieur de 1 à 6 atomes de carbone
- un radical trifluorométhyle
- un radical O-alkyle inférieur de 1 à 6 atomes de carbone

$R_1R_2$ représentent un atome d'oxygène
$R_3$,$R_4$ représentent indépendamment l'atome d'hydrogène ou un radical alkyle inférieur de 1 à 6 atomes de carbone.

**3.** Dérivés selon la revendication 1 ou 2 caractérisés en ce que le cycle B représente un radical phényle.

**4.** Dérivés selon la revendication 1 ou 2 caractérisés en ce que le cycle A représente un radical phényle ou pyridyle.

**5.** Dérivés selon l'une quelconque des revendications 1 à 4 caractérisés en ce que $X_1$ représente le groupement 4-$SO_2CH_3$ ou le groupement 4-$SO_2NH_2$ et $X_2$ représente l'atome d'hydrogène.

**6.** Dérivés selon l'une quelconque des revendications 1 à 5 caractérisés en ce que $Y_1$ représente l'atome de fluor, l'atome de chlore ou un radical méthyle et $Y_2$ représente l'atome d'hydrogène, l'atome de fluor ou l'atome de chlore.

**7.** Dérivés selon l'une quelconque des revendications 1 à 6 caractérisés en ce que $R_1R_2$ représente l'atome d'oxygène et $R_3$ et $R_4$ représentent chacun un atome d'hydrogène.

**8.** Dérivés selon la revendication 1, caractérisés en ce qu'ils sont choisis parmi les composés de formule :

(E)-3-[1-(4-fluorophényl)-1-(4-méthanesulfonylphényl)méthylidène]-dihydrofuran-2-one

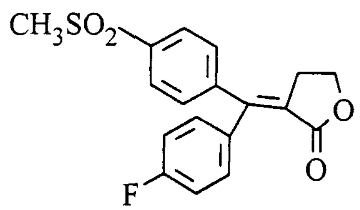

(Z)-3-[1-(4-chlorophényl)-1-(4-méthanesulfonylphényl)méthylidène]-dihydrofuran-2-one

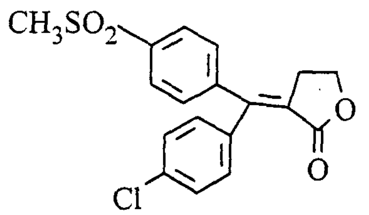

(Z)-3-[1-(3,4-dichlorophényl)-1-(4-méthanesulfonylphényl)méthylidène]-dihydrofuran-2-one

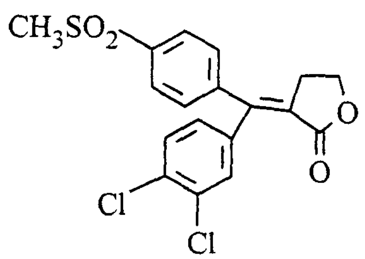

(Z)-3-[1-(6-chloropyridin-3-yl)-1-(4-méthanesulfonylphényl)méthylidène]-dihydro-furan-2-one

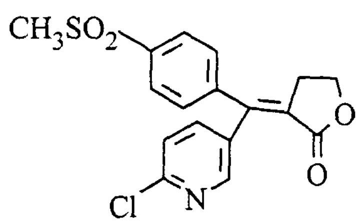

(Z)-4-[(4-chlorophényl)-(2-oxo-dihydro-furan-3-ylidène)-méthyl]-benzène sulfonamide

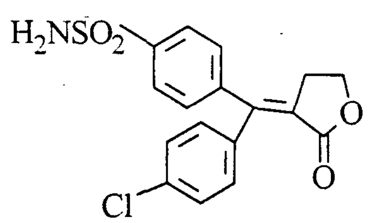

(Z)-4-[(3-fluoro-4-méthylphényl)-(2-oxo-dihydro-furan-3-ylidène)-méthyl]-benzènesulfonamide

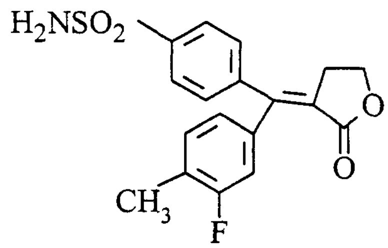

**9.** Procédé de préparation des composés de formule (I) selon l'une quelconque des revendications 1 à 8 caractérisé en ce qu'il comprend la réaction d'une cétone de formule

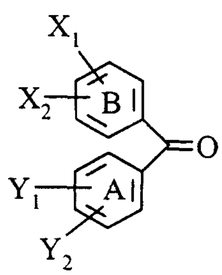

dans laquelle A, B, $X_1$, $X_2$, $Y_1$ et $Y_2$ sont tels que définis à la revendication 1, avec un succinate d'alkyle de formule

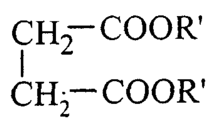

dans laquelle R' est un radical alkyle inférieur de 1 à 6 atomes de carbone, optimalement le radical éthyle,selon les condition de la réaction de condensation de Stobbe en présence d'un alcoolate, en particulier le terbutylate de sodium ou de potassium ou d'un hydrure comme l'hydrure de sodium dans un solvant alcoolique comme le terbutanol ou un solvant aromatique comme le toluène, le choix de l'agent de condensation et du solvant pouvant permettre d'orienter la réaction vers un isomère particulier,
pour conduire aux dérivés esters acides de formule

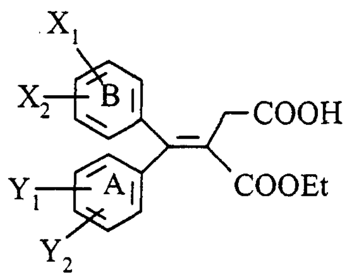

dans laquelle A, B, $X_1$, $X_2$, $Y_1$ et $Y_2$ sont tels que définis à la revendication 1 ; la réduction sélective de la fonction acide des dérivés esters acides précités, par exemple par action d'un borane dans le tétrahydrofurane pour conduire aux esters alcools de formule

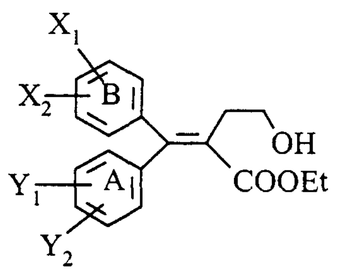

dans laquelle A, B, $X_1$, $X_2$, $Y_1$ et $Y_2$ sont tels que définis à la revendication 1, puis la cyclisation de ces esters alcools par chauffage par exemple dans le toluène en présence d'acide paratoluènesulfonique.

**10.** Composition pharmaceutique, caractérisée en ce qu'elle comprend une quantité pharmaceutiquement efficace d'au moins un composé de formule (I) tel que défini à l'une quelconque des revendications 1 à 8, éventuellement incorporé dans un excipient, véhicule ou support pharmaceutiquement acceptable.

**11.** Composition pharmaceutique à activité anti-inflammatoire et antalgique, caractérisée en ce qu'elle renferme une quantité pharmaceutiquement efficace d'un composé de formule (I) tel que défini à l'une quelconque des revendications 1 à 8, éventuellement incorporé dans un excipient, véhicule ou support pharmaceutiquement acceptable.

**12.** Composition pharmaceutique utile dans la prévention du cancer, en particulier l'adénocarcinome du colon, la prévention des maladies neurodégénératives particulièrement la maladie d'Alzheimer, la prévention du stroke, l'épilepsie et la prévention du travail utérin prématuré, caractérisée en ce qu'elle contient une quantité pharmaceutiquement efficace d'au moins un composé de formule (I) tel que défini à l'une quelconque des revendications 1 à 8, éventuellement incorporé dans un excipient, véhicule ou support pharmaceutiquement acceptable.

**13.** Composition pharmaceutique selon la revendication 10 ou 11 caractérisée en ce qu'elle se présente sous forme de gélules, de comprimés dosés de 1 mg à 1000 mg ou sous forme de préparations injectables dosées de 0,1 mg à 500 mg.

**Patentansprüche**

**1.** Diarylmethylidenfuranyl-Derivate, dadurch gekennzeichnet, dass sie durch die allgemeine Formel (I) dargestellt sind:

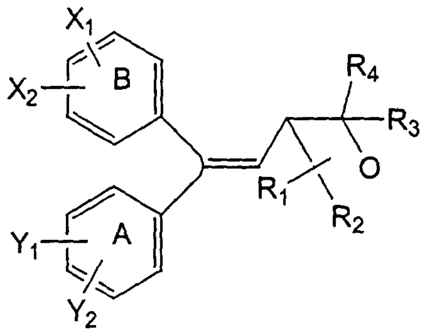

(Formel I)

worin gilt:

die Ringe A und B stellen, unabhängig von einander, dar:

- einen Phenylrest,
- einen Naphthylrest,
- einen von einem 5- bis 6-gliedrigen Heterocyclus mit 1 bis 4 Heteroatomen abgeleiteten Rest,
- einen von einem gesättigten Kohlenwasserstoffring mit 3 bis 7 Kohlenstoffatomen abgeleiteten Rest,

wobei mindestens einer der Ringe A und B zwingend einen Phenylrest darstellt, mindestens einer der Substituenten $X_1$, $X_2$, $Y_1$ oder $Y_2$ stellt zwingend dar:

- eine $-S(O)_n-R$-Gruppierung, worin n eine ganze Zahl gleich 0, 1 oder 2 und R ein Niedrigalkylrest mit 1 bis 6 Kohlenstoffatomen oder ein Halogenniedrigalkylrest mit 1 bis 6 Kohlenstoffatomen sind,
- eine $-SO_2NH_2$-Gruppierung, die sich in p-Position am durch A oder B dargestellten Phenylrest befindet,

die anderen Substituenten stellen, unabhängig von einander, dar:

- das Wasserstoffatom,
- ein Halogenatom,
- einen Niedrigalkylrest mit 1 bis 6 Kohlenstoffatomen,
- einen Trifluormethylrest,
- einen 0-Niedrigalkylrest mit 1 bis 6 Kohlenstoffatomen,

oder

$X_1$ und $X_2$ oder $Y_1$ und $Y_2$ stellen auch eine Methylendioxi-Gruppierung dar,

$R_1$, $R_2$, $R_3$ und $R_4$ stellen, unabhängig von einander, dar:

- das Wasserstoffatom,
- ein Halogenatom,
- einen Niedrigalkylrest mit 1 bis 6 Kohlenstoffatomen,

- einen Halogenniedrigalkylrest mit 1 bis 6 Kohlenstoffatomen,
- einen aromatischen Rest, ausgewählt aus der Gruppe, umfassend einen Phenyl-, Naphthyl-, Thienyl-, Furyl- oder einen Pyridylrest,

oder

$R_1R_2$ oder $R_3R_4$ stellen auch ein Sauerstoffatom dar,

oder $R_1$, $R_2$ oder $R_3$, $R_4$ bilden zusammen mit dem Kohlenstoffatom, an das sie gebunden sind, auch einen gesättigten Kohlenwasserstoffring mit 3 bis 7 Kohlenstoffatomen.

**2.** Derivate der Formel (I) gemäß Anspruch 1, dadurch gekennzeichnet, dass gilt:

die Ringe A und B stellen, unabhängig von einander, einen Rest dar:

- Phenyl
- Naphthyl
- Pyridyl
- Furyl
- Thienyl
- Cyclohexyl,

mindestens einer der Substituenten $X_1$, $X_2$, $Y_1$ oder $Y_2$ stellt zwingend eine $-SCH_3$-, $-SO_2CH_3$ oder $-SO_2NH_2$-Gruppierung dar,

die anderen Substituenten stellen, unabhängig von einander, dar:

- das Wasserstoffatom,
- ein Halogenatom,
- einen Niedrigalkylrest mit 1 bis 6 Kohlenstoffatomen,
- einen Trifluormethylrest,
- einen O-Niedrigalkylrest mit 1 bis 6 Kohlenstoffatomen,

$R_1R_2$ stellen ein Sauerstoffatom dar,

$R_3$ und $R_4$ stellen, unabhängig von einander, das Wasserstoffatom oder einen Niedrigalkylrest mit 1 bis 6 Kohlenstoffatomen dar.

**3.** Derivate gemäß Anspruch 1 oder 2,
dadurch gekennzeichnet, dass der Ring B einen Phenylrest darstellt.

**4.** Derivate gemäß Anspruch 1 oder 2, dadurch gekennzeichnet, dass der Ring A einen Phenyl- oder Pyridylrest darstellt.

**5.** Derivate gemäß einem der Ansprüche 1 bis 4,
dadurch gekennzeichnet, dass $X_1$ die $4-SO_2CH_3$- oder die $4-SO_2NH_2$-Gruppierung und $X_2$ das Wasserstoffatom darstellen.

**6.** Derivate gemäß einem der Ansprüche 1 bis 5, dadurch gekennzeichnet, dass $Y_1$ das Fluoratom, das Chloratom oder einen Methylrest und $Y_2$ das Wassesrstoffatom, das Fluor- oder das Chloratom darstellen.

**7.** Derivate gemäß einem der Ansprüche 1 bis 6,
dadurch gekennzeichnet, dass $R_1R_2$ das Sauerstoffatom und $R_3$ und $R_4$ jeweils ein Wasserstoffatom darstellen.

**8.** Derivate gemäß Anspruch 1, dadurch gekennzeichnet, dass sie aus den Verbindungen der Formel ausgewählt sind:

(Z)-3- [1- (4-Fluorphenyl)-1-(4-methansulfonylphenyl)methyliden]dihydrofuran-2-on:

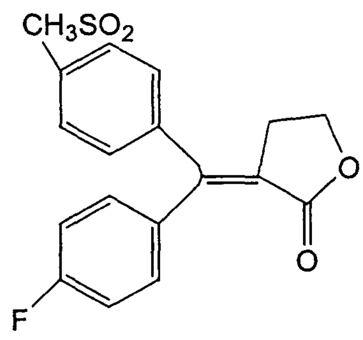

(Z)-3-[1-(4-Chlorphenyl)-1-(4-methansulfonylphenyl)methyliden]dihydrofuran-2-on:

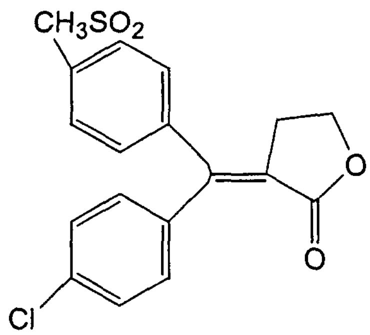

(Z)-3-[1-(3,4-Dichlorphenyl)-1-(4-methansulfonylphenyl)methyliden]dihydrofuran-2-on:

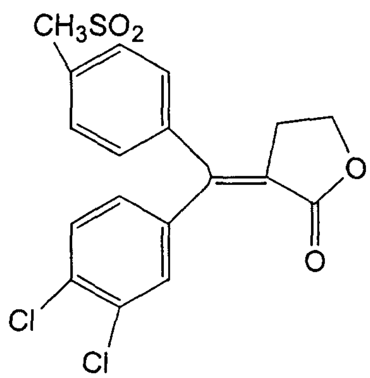

(Z)-3-[1-(6-Chlorpyridin-3-yl)-1-(4-methansulfonylphenyl)methyliden]dihydrofuran-2-on:

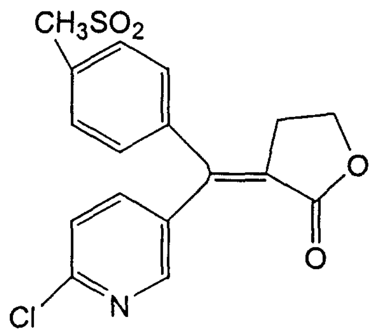

(Z)-4-[(4-Chlorphenyl)-(2-oxodihydrofuran-3-yliden)methyl)benzolsulfonamid:

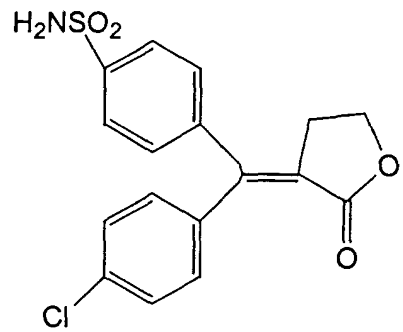

(Z)-4-[(3-Fluor-4-methylphenyl)-(2-oxodihydrofuran-3-yliden)methyl)benzolsulfonamid:

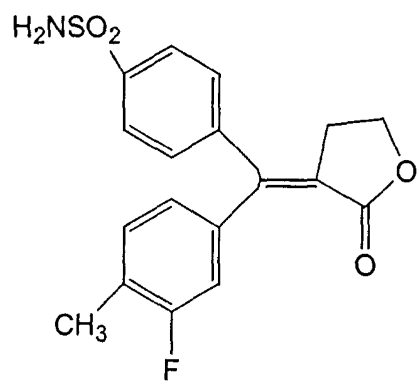

**9.** Verfahren zur Herstellung von Verbindungen der Formel (I) gemäß einem der Ansprüche 1 bis 8, dadurch gekennzeichnet, dass man ein Keton der Formel:

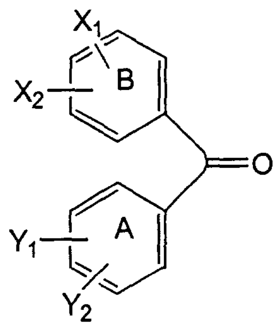

worin A, B, $X_1$, $X_2$, $Y_1$ und $Y_2$ wie in Anspruch 1 definiert sind, mit einem Alkylsuccinat der Formel:

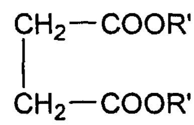

worin R' ein Niedrigalkylrest mit 1 bis 6 Kohlenstoffatomen, am besten der Ethylrest, ist, gemäß den Bedingungen der Stobbe-Kondensationsreaktion in Gegenwart eines Alkoholats, insbesondere von Natrium- oder Kalium-t-butylat, oder eines Hydrids wie von Natriumhydrid in einem alkoholischen Lösungsmittel wie t-Butanol oder in einem aromatischen Lösungsmittel wie Toluol, wobei die Wahl des Kondensations- und Lösungsmittels die Reaktion in Richtung auf ein besonderes Isomer zu lenken ermöglicht, zur Reaktion bringt, um saure Halbesterderivate der Formel zu ergeben:

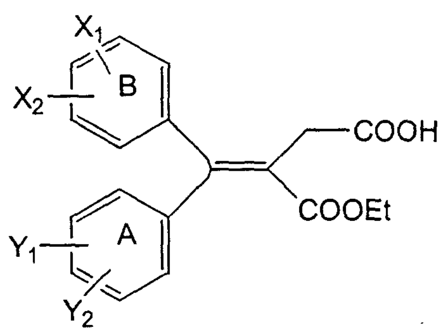

worin A, B, $X_1$, $X_2$, $Y_1$ und $Y_2$ wie in Anspruch 1 definiert sind, worauf man die Säure-Funktion der vorgenannten Halbesterderivate beispielsweise durch Einwirkung eines Borans in Tetrahydrofuran selektiv reduziert, um die Esteralkohole der Formel zu ergeben:

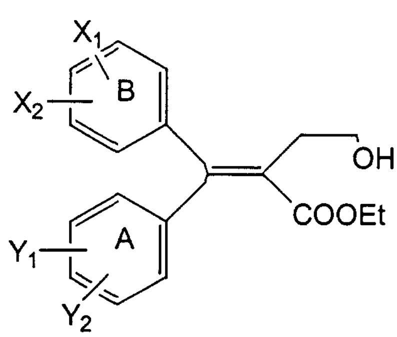

worin A, B, $X_1$, $X_2$, $Y_1$ und $Y_2$ wie in Anspruch 1 definiert sind, und man dann diese Esteralkohole durch Erhitzen

beispielsweise in Toluol in Gegenwart von p-Toluolsulfonsäure zyklisiert.

**10.** Pharmazeutische Zusammensetzung, dadurch gekennzeichnet, dass sie eine pharmazeutisch wirksame Menge mindestens einer in einem der Ansprüche 1 bis 8 definierten Verbindung der Formel (I) umfasst, die gegebenenfalls in einen pharmazeutisch zulässigen Exzipient, Träger oder eine entsprechende Trägergrundlage eingebracht sind.

**11.** Pharmazeutische Zusammensetzung mit entzündungshemmender und antalgetischer Aktivität, dadurch gekennzeichnet, dass sie eine pharmazeutisch wirkame Menge einer in einem der Ansprüche 1 bis 8 definierten Verbindung der Formel (I) einschließt, die gegebenenfalls in einen pharmazeutisch zulässigen Exzipient, Träger oder eine entsprechende Trägergrundlage eingebracht sind.

**12.** Pharmazeutische Zusammensetzung zur Verhütung von Krebs, insbesondere eines Adenocarcinoms des Darms, zur Verhütung neurodegenerativer Krankheiten und insbesondere der Alzheimer-Krankheit, zur Verhütung eines Schlaganfalls und von Epilepsie sowie zur Verhütung einer vorzeitigen Gebärmuttertätigkeit,
dadurch gekennzeichnet, dass sie eine pharmazeutisch wirksame Menge mindestens einer in einem der Ansprüche 1 bis 8 definierten Verbindung der Formel (I) enthält, die gegebenenfalls in einen pharmazeutisch zulässigen Exzipient, Träger oder eine entsprechende Trägergrundlage eingebracht sind.

**13.** Pharmazeutische Zusammensetzung gemäß Anspruch 10 oder 11,
dadurch gekennzeichnet, dass sie in Form von Gelatinekapseln oder Tabletten mit Dosismengen von 1 bis 1000 mg oder in Form injizierbarer Zubereitungen mit Dosismengen von 0,1 bis 500 mg vorliegt.

**Claims**

**1.** Diarylmethylidenefuran derivatives, characterised in that they are of general formula (I):

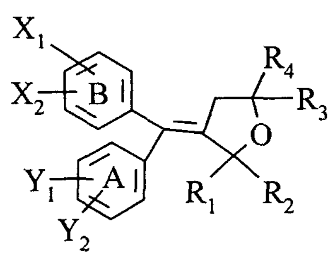

Formula (I)

in which:

the rings A and B independently are:

- a phenyl radical,
- a naphthyl radical,
- a radical derived from a heterocycle comprising 5 to 6 members and possessing from 1 to 4 heteroatoms, or
- a radical derived from a saturated hydrocarbon ring having from 3 to 7 carbon atoms;

at least one of the rings A and B necessarily representing a phenyl radical,
at least one of the substituents $X_1$, $X_2$, $Y_1$ or $Y_2$ is necessarily:

- an $-S(O)_n-R$ group, in which n is an integer equal to 0, 1 or 2 and R is a lower alkyl radical having 1 to 6 carbon atoms or a lower haloalkyl radical having 1 to 6 carbon atoms, or
- an $-SO_2NH_2$ group;
  and is located in the para position on the phenyl radical represented by A or B,

the others independently being:

- a hydrogen atom,
- a halogen atom,
- a lower alkyl radical having 1 to 6 carbon atoms,
- a trifluoromethyl radical, or
- a lower O-alkyl radical having 1 to 6 carbon atoms, or even

$X_1$ and $X_2$ or $Y_1$ and $Y_2$ are a methylenedioxy group; and
$R_1$, $R_2$, $R_3$ and $R_4$ independently are:

- a hydrogen atom,
- a halogen atom,
- a lower alkyl radical having 1 to 6 carbon atoms,
- a lower haloalkyl radical having 1 to 6 carbon atoms, or
- an aromatic radical selected from the group consisting of phenyl, naphthyl, thienyl, furyl or pyridyl; or even

$R_1R_2$ or $R_3R_4$ are an oxygen atom, or even
$R_1$,$R_2$ or $R_3$,$R_4$, together with the carbon atom to which they are attached, form a saturated hydrocarbon ring having from 3 to 7 carbon atoms.

**2.** Derivatives of formula (I) according to claim 1, characterised in that:

the rings A and B independently are:

- a phenyl radical,
- a naphthyl radical,
- a pyridyl radical,
- a furyl radical,
- a thienyl radical, or
- a cyclohexyl radical;

at least one of the substituents $X_1$, $X_2$, $Y_1$ or $Y_2$ is necessarily an $-SCH_3$, $-SO_2CH_3$ or $-SO_2NH_2$ group,
the others independently being:

- a hydrogen atom,
- a halogen atom,
- a lower alkyl radical having 1 to 6 carbon atoms,
- a trifluoromethyl radical, or
- a lower O-alkyl radical having 1 to 6 carbon atoms;

$R_1R_2$ are an oxygen atom; and
$R_3$,$R_4$ independently are a hydrogen atom or a lower alkyl radical having 1 to 6 carbon atoms.

**3.** Derivatives according to claim 1 or 2, characterised in that the ring B is a phenyl radical.

**4.** Derivatives according to claim 1 or 2, characterised in that the ring A is a phenyl radical or a pyridyl radical.

**5.** Derivatives according to any one of claims 1 to 4, characterised in that $X_1$ is a 4-$SO_2CH_3$ group or a 4-$SO_2NH_2$ group, and $X_2$ is a hydrogen atom.

**6.** Derivatives according to any one of claims 1 to 5, characterised in that $Y_1$ is a fluorine atom, a chlorine atom or a methyl radical, and $Y_2$ is a hydrogen atom, a fluorine atom or a chlorine atom.

**7.** Derivatives according to any one of claims 1 to 6, characterised in that $R_1R_2$ are an oxygen atom and $R_3$ and $R_4$ are each a hydrogen atom.

**8.** Derivatives according to claim 1, characterised in that they are selected from the compounds of formulae:

(E)-3-[1-(4-fluorophenyl)-1-(4-methanesulphonylphenyl)methylidene]-dihydrofuran-2-one

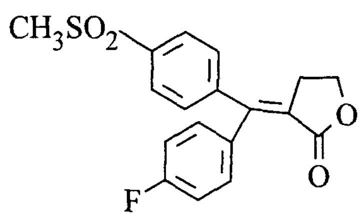

(Z)-3-[1-(4-chlorophenyl)-1-(4-methanesulphonylphenyl)methylidene]-dihydrofuran-2-one

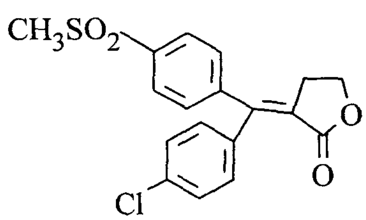

(Z)-3-[1-(3,4-dichlorophenyl)-1-(4-methanesulphonylphenyl)methylidene]-dihydro-furan-2-one

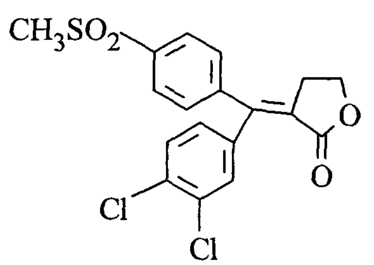

(Z)-3-[1-(6-chloropyridin-3-yl)-1-(4-methanesulphonylphenyl)methylidene]-dihydro-furan-2-one

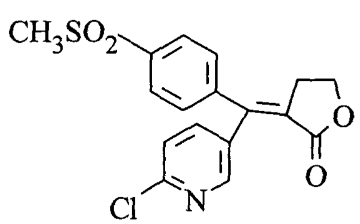

(Z)-4-[(4-chlorophenyl)-(2-oxo-dihydro-furan-3-ylidene)-methyl]-benzene sulphonamide

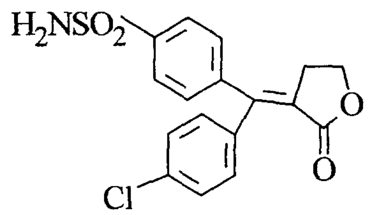

(Z)-4-[(3-fluoro-4-methylphenyl)-(2-oxo-dihydro-furan-3-ylidene)-methyl]-benzenesulphonamide

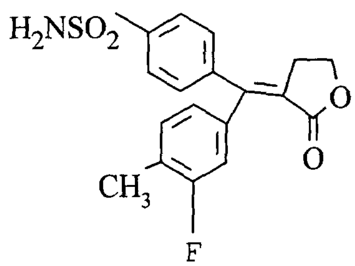

**9.** A process for the preparation of the compounds of formula (I) according to any one of claims 1 to 8, characterised in that it comprises the reaction of a ketone of formula:

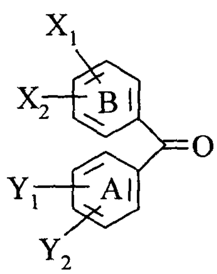

in which A, B, $X_1$, $X_2$, $Y_1$ and $Y_2$ are such as defined in claim 1,
with an alkyl succinate of the formula

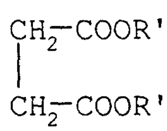

in which R' is a lower alkyl radical having 1 to 6 carbon atoms, optimally an ethyl radical, according to the conditions of the Stobbe condensation reaction, in the presence of an alkoxide, in particular sodium or potassium tert-butoxide, or a hydride such as sodium hydride in an alcohol solvent such as tert-butanol or an aromatic solvent such as toluene, the choice of the condensation agent and the solvent making it possible to orient the reaction towards a particular isomer, to give the acid ester derivatives of formula:

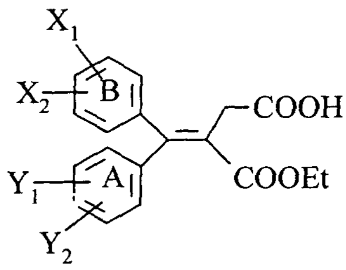

in which A, B, $X_1$, $X_2$, $Y_1$ and $Y_2$ are such as defined in claim 1; the selective reduction of the acid function of the above ester acids, for example by the action of a borane in tetrahydrofuran, leading to the ester alcohols of the formula:

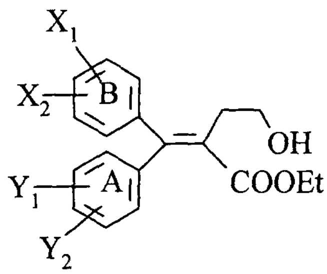

in which A, B, $X_1$, $X_2$, $Y_1$ and $Y_2$ are such as defined in claim 1, and then cyclising these ester alcohols by heating for example in toluene in the presence of paratoluenesulphonic acid.

**10.** A pharmaceutical composition, characterised in that it comprises a pharmaceutically effective amount of at least one compound of formula (I) as defined in any one of claims 1 to 8, optionally incorporated in a pharmaceutically acceptable excipient, vehicle or carrier.

**11.** A pharmaceutical composition with anti-inflammatory and analgesic activity, characterised in that it contains a pharmaceutically effective amount of a compound of formula (I) as defined in any one of claims 1 to 8, optionally incorporated in a pharmaceutically acceptable excipient, vehicle or carrier.

**12.** A pharmaceutical composition useful in the prevention of cancer, in particular adenocarcinoma of the colon, in the prevention of neurodegenerative diseases, especially Alzheimer's disease, in the prevention of stroke and epilepsy, and in the prevention of premature labour, characterised in that it contains a pharmaceutically effective amount of a compound of formula (I) as defined in any one of claims 1 to 8, optionally incorporated in a pharmaceutically acceptable excipient, vehicle or carrier.

**13.** A pharmaceutical composition according to claim 10 or 11, characterised in that it is in the form of gelatine capsules or tablets containing a dose of 1 mg to 1000 mg, or in the form of injectable preparations containing a dose of 0.1 mg to 500 mg.